(19) Europäisches Patentamt
European Patent Office
Office européen des brevets

(11) EP 0 837 859 B1

(12) **EUROPEAN PATENT SPECIFICATION**

(45) Date of publication and mention of the grant of the patent:
**13.08.2003 Bulletin 2003/33**

(21) Application number: **96921326.3**

(22) Date of filing: **17.06.1996**

(51) Int Cl.$^7$: **C07D 405/14**, A61K 31/41

(86) International application number:
**PCT/US96/09242**

(87) International publication number:
**WO 97/000255 (03.01.1997 Gazette 1997/02)**

(54) **TETRAHYDROFURAN ANTIFUNGALS**

ANTIFUNGALE TETRAHYDROFURANE

TETRAHYDROFURANES ANTI-MYCOTIQUES

(84) Designated Contracting States:
**AT BE CH DE DK ES FI FR GB GR IE IT LI LU NL PT SE**
Designated Extension States:
**LT LV**

(30) Priority: **19.06.1995 US 305 P**
**05.02.1996 US 596852**

(43) Date of publication of application:
**29.04.1998 Bulletin 1998/18**

(73) Proprietor: **SCHERING CORPORATION**
**Kenilworth, New Jersey 07033-0530 (US)**

(72) Inventors:
- **LOVEY, Raymond, G.**
**West Caldwell, NJ 07006 (US)**
- **GIRIJAVALLABHAN, Viyyoor, M.**
**Parsippany, NJ 07054 (US)**
- **SAKSENA, Anil, K.**
**Upper Montclair, NJ 07043 (US)**
- **PIKE, Russell, E.**
**Stanhope, NJ 07874 (US)**
- **BENNET, Frank**
**Piscataway, NJ 08854 (US)**

(74) Representative: **Ritter, Stephen David et al**
**Mathys & Squire**
**100 Gray's Inn Road**
**London WC1X 8AL (GB)**

(56) References cited:
**EP-A- 0 539 938** **WO-A-95/17407**
**WO-A-95/19983**

- **SCHRÖDER Eberhard et al, Arzneimittelchemie I, Stuttgart, Georg Thieme Verlag 1976, p.24-39**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 837 859 B1

## Description

BACKGROUND OF THE INVENTION

This invention relates to tetrahydrofuran antifungals, hydroxylic derivatives, esters, ethers and salts thereof, pharmaceutical compositions containing them, and methods of treating and/or preventing antifungal infections in hosts, including warm-blooded animals, especially humans with such tetrahydrofuran antifungals.

There is a need for broad-spectrum antifungal agents having increased solubility and having favorable activity profile for treating systemic fungal infections, especially Aspergillus, Candida, Cyrptococcus and other opportunistic fungal infections.

EP 0 539 938 relates to antifungal compounds which include a 2,4-substituted tetrahydrofuran moiety.

The present invention provides a compound represented by formula IIIa

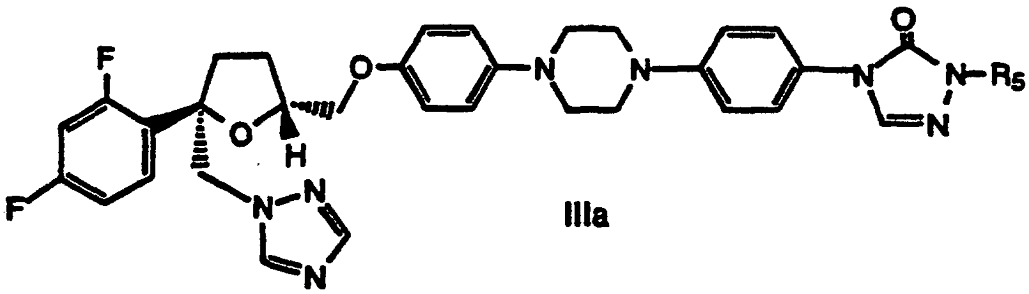

IIIa

wherein $R_5$ is

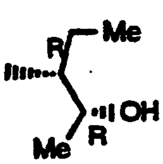 , 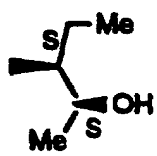 , 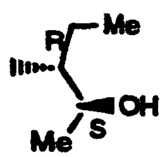 ,

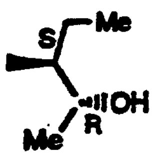 , 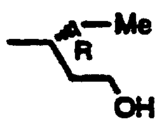 , 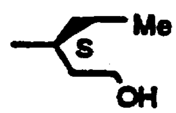 ,

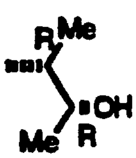 , 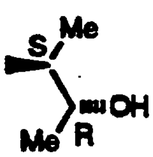 , 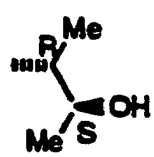 ,

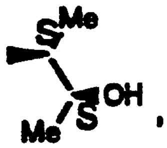,
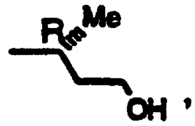,
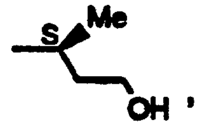,

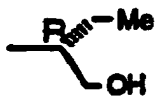 or 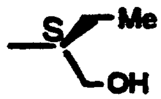

or an ester thereof or a pharmaceutically acceptable salt thereof,

wherein the ester is a (a) polyether ester, (b) phosphate ester, (c) heterocyclic esters (d) alkanoate and alkenoate ester, (e) amino acid ester (f) carboxylic acid ester, (g) carbonic and carbamic acid ester, (h) sulfate ester.

In another aspect of the present invention there is provided a compound represented by the formula IVa.

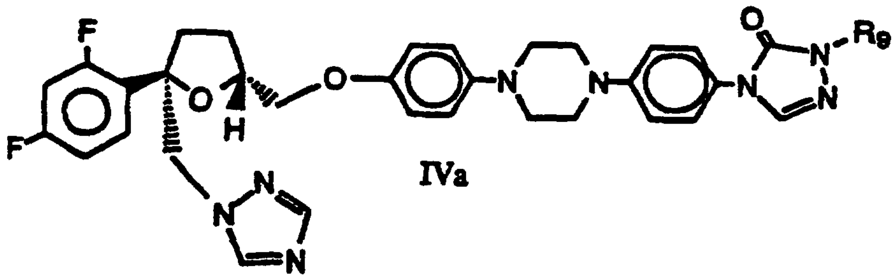

IVa

wherein $R_9$ =

$$-\overset{*}{C}H(C_2H_5)CH(R_6)CH_3 \text{ or } -\overset{*}{C}H(CH_3)CH(R_6)CH_3$$

wherein $R_6$ is OH, or an ester thereof or a pharmaceutically acceptable salt thereof, wherein said ester is as defined above.

Exemplary of the compounds of the invention are the following:

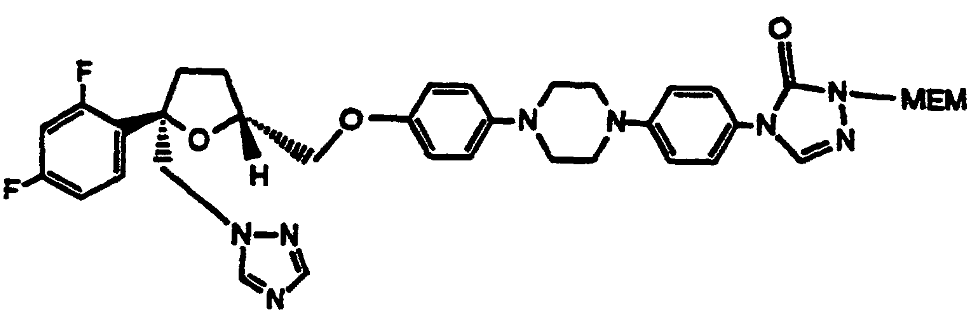

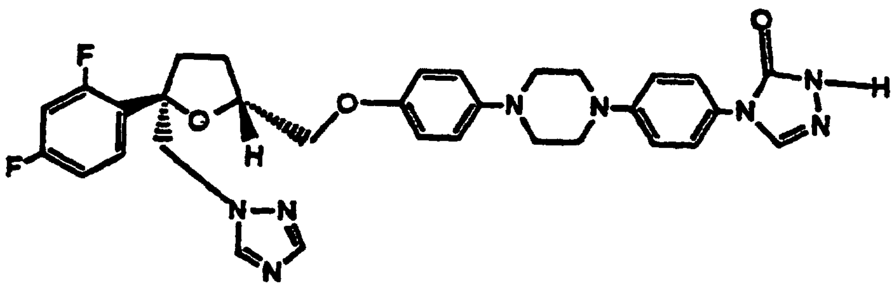

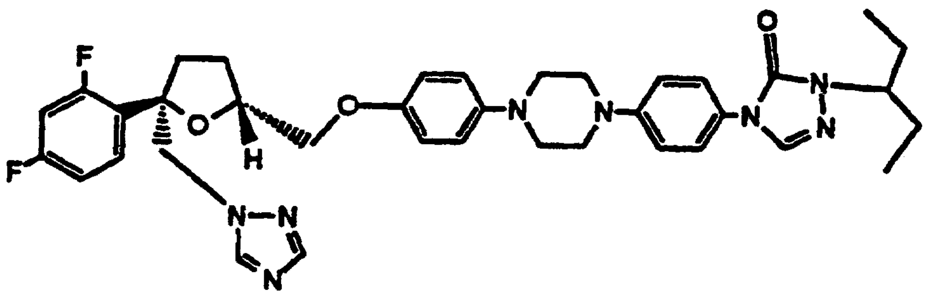

and

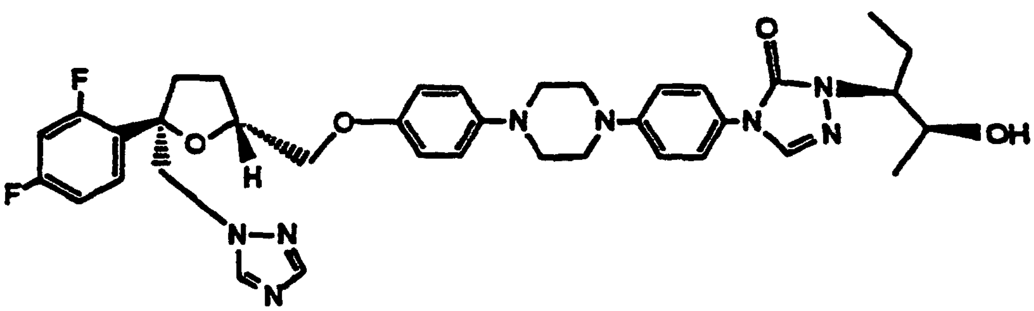

or a pharmaceutically acceptable salt thereof.
The most preferred compound of the invention is

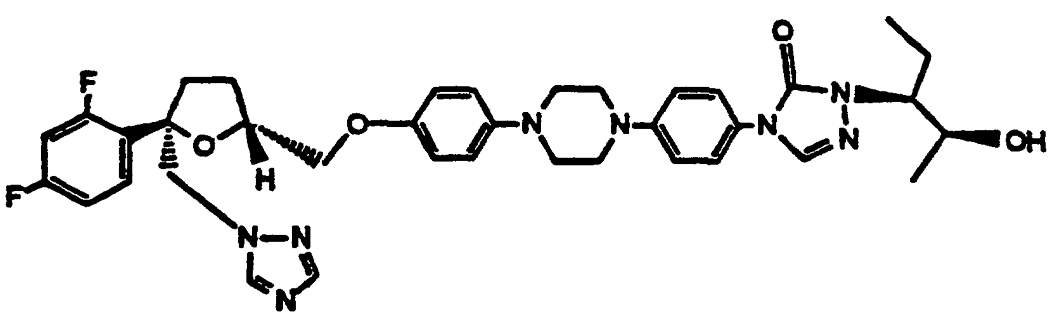

or a pharmaceutically acceptable salt thereof; or an α-amino acid ester thereof; or a pharmaceutically acceptable salt of an α-amino acid ester thereof.

The hydroxy compound shown just above is preferred for oral use.

The invention further relates to amino acid esters of the type shown just below:

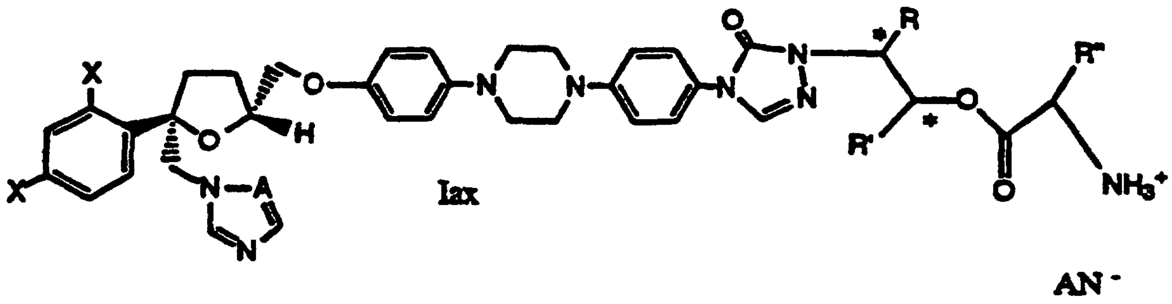

Iax

wherein X is independently selected from the group consisting of F, Cl Br, and $CF_3$;

A is N or CH;
R is H, $CH_3$, or $C_2H_5$,:
R' is H, $CH_3$, $C_2H_5$,
$AN^-$ is an anion such as Cl- or $C_3H_5O_3^-$ ;
R" is H, $CH_3$, $CH_2OR'''$, $CH(OR''')CH_3$; $CH_2SR'''$,

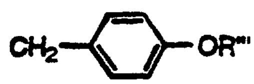
,

$CH_2CONH_2$, $CH_2CH_2$,$CONH_2$,

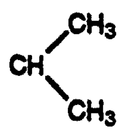
,

$CH(CH_3)CH_2CH_3$,

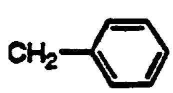
,
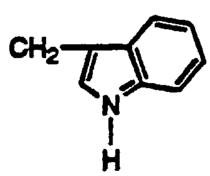
,

$CH_2CH_2SCH_3$,

$CH_2COO^-\ M^+$, $CH_2CH_2COO^-\ M^+$, $CH_2CH_2CH_2CH_2NH_3{}^+\ AN^-$

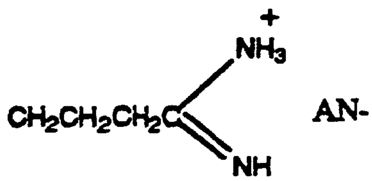

,

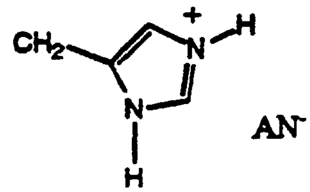

$M^+$ is a metal cation such as $Na^+$; and
R''' is H, phosphate ester, sulphate ester and proline ester.

An α-amino acid ester thereof which is shown just above is preferred for intravenous use.

DETAILED DESCRIPTION OF THE INVENTION

The term "($C_3$-$C_8$) alkyl group substituted by one or two hydroxy moieties", as used herein means straight and branched chain alkyl groups of three to eight carbons including but not limited to methyl, ethyl, n- and iso-propyl, n-, sec-, iso- and tert-butyl, n-, sec-, iso-, tert and neo-pentyl n-, sec-, iso-, tert- and neo-hexyl, n-, sec-, iso-, tert- and neo-heptyl, n, sec- iso, tert-and neo octyl, substituted by one or two hydroxy moieties and includes R and S stereoisomers of such ($C_3$-$C_8$) alkyl groups.

The term *($C_1$-$C_3$) alkyl substituted by one hydroxy moiety" means $-CH_2OH$,

$$-\overset{*}{C}H(OH)CH_3,$$

$$-CH_2CH_2OH,$$

$$-\overset{*}{C}H(OH)C_2H_5,$$

$$-\overset{*}{C}H_2CH(OH)CH_3,$$

and $-(CH_2)_3$-OH wherein the carbons with the asterisk(*) have the R or S absolute configuration.

The term "hydroxy-substituted $C_4$ or $C_5$ alkyl group" means

$$-\overset{*}{C}H(C_2H_5)\overset{*}{C}H(OH)CH_3,\ -\overset{*}{C}H(C_2H_5)CH_2CH_2OH,\ -(CH_2)_2-\overset{*}{C}H(OH)C_2H_5,$$

$$-\overset{*}{C}H(CH_3)\overset{*}{C}H(OH)CH_3,\ -\overset{*}{C}H(CH_3)\overset{*}{C}H(OH)CH_3 \text{ or } -\overset{*}{C}H(C_2H_5)CH_2OH$$

wherein each carbon with the asterisk (*) has the R or S absolute configuration.

The term "MEM" means methoxyethoxymethyl.

The term "group convertible in vivo into OH" means a group transformable in vivo by e.g. hydrolysis and/or by an enzyme, e.g. an esterase into a hydroxyl group. Such groups include polyether esters, phosphate esters, sulfate esters, heterocyclic esters, alkanoate esters, alkenoate esters, carbonate esters, amino acid esters, carbamate esters, and acid esters. Preferred groups convertible in vivo into a hydroxyl group are the polyether esters, phosphate esters and amino acid esters.

The term "ether" as it relates to protective groups in the syntheses given herein, means ($C_1$-$C_6$) alkoxy or aryl ($C_1$-$C_6$) alkoxy which are conveniently made by the well known Williamson Synthesis of ethers. Typically suitable ether groups include methoxy and benzoxy.

The term "esters" means (a) polyether esters (b) phosphate esters (c) heterocyclic esters (d) alkanoate and

alkenoate esters (e) amino-acid esters (f) carboxylic acid esters (g) carbonic and carbamic acid esters and (h) sulfate esters.

The term "polyether esters" as used herein means those polyether esters represented by the formula

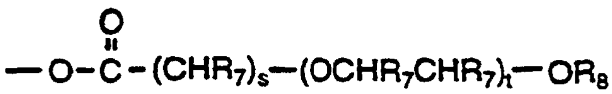

wherein $R_7$ is a ($C_1$-$C_6$) straight or branched chain alkyl. Where more than one $R_7$ appear in a molecule, each $R_7$ may be the same or different from the others. "s" is an integer from 1 to 6, preferably s = 1 to 3 and more preferably s = 1; t is an integer from 1 to 200; $R_8$ is $R_7$ or $—(CHR_7)_s$-$CO_2R_7$ ; preferably $R_8$ is $CH_3$ or $C_2H_5$ or $—CH_2CO_2H$ or $—CH_2CO_2CH_3$. Typically suitable polyether esters include $-COCH_2O(CH_2CH_2O)_1CH_3$; $—COCH_2O(CH_2CH_2O)_2CH_3$, and $-COCH_2O(CH_2CH_2O)_3CH_3$.

The term "phosphate esters" as used herein means those phosphate acids esters represented by the formula

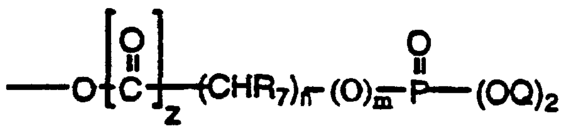

or

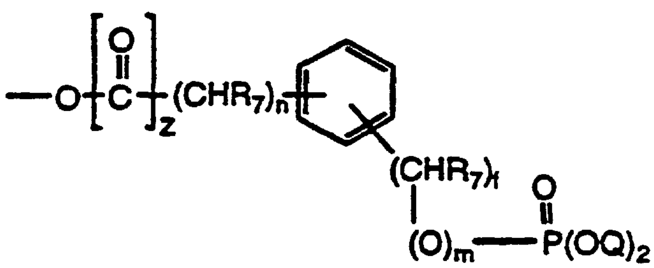
,

wherein z is 0 or 1; $R_7$ is as defined herein above and preferably is H, and where more than one $R_7$ appears in a molecule, each $R_7$ may be the same or different from the others; n and f are independently an integer from 0 to 6, m is 0 or 1 and Q is H, $CH_2Ar$ or

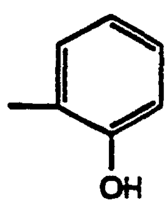

and wherein Ar is phenyl, phenyl substituted by halo, especially chloro and fluoro, or by nitro, cyano and trihalomethyl especially trifluoromethyl.

Typically suitable phosphate acids and esters include

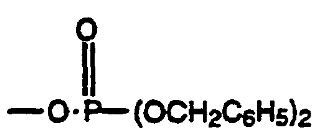
,

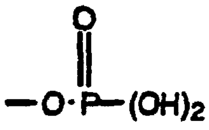, 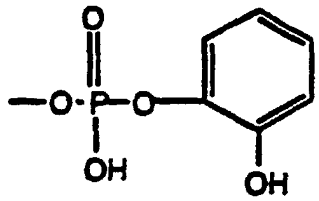, 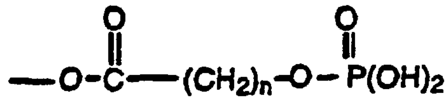

wherein m= n = 1 to 4; or

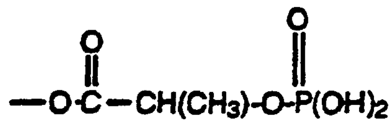

and pharmaceutically acceptable salts thereof.

The term "heterocyclic ester" as used herein means heterocyclic esters represented by the formula

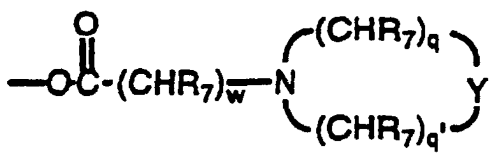

wherein $R_7$ is as defined herein above, w is an integer of from 1 to 5 preferably w is 1 to 3; q and q' are independently 1 to 4, and q + q' are preferably equal to 2, 3, 4, or 5, and Y is $CHR_7$, -O-, NH, $NR_7$, S, SO or $SO_2$

Typically suitable heterocyclic esters include

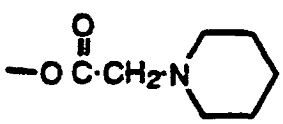, 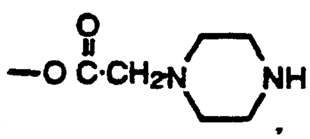, 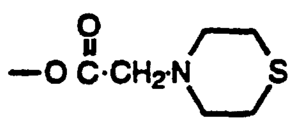,

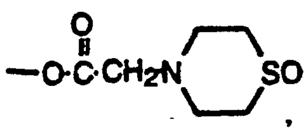, 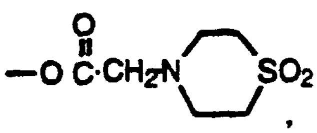, 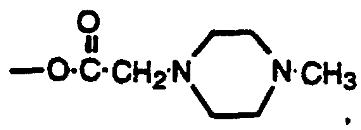,

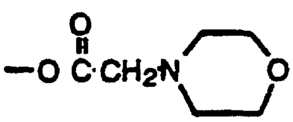, and 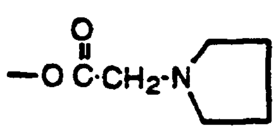.

The term "alkanoate and alkenoate esters' as used herein means straight or branched chain alkanoate or alkenoate groups optionally substituted by a hydroxy or ether moiety or mixtures of such alkanoates or alkenoates.

Preferred alkanoate esters include acetate to decanoate, especially acetate to butanoate. Preferred hydroxy substituted alkanoate ester include $C_1$ to $C_8$ alkanoate substituted one hydroxy moiety or one $C_1$-$C_6$ alkoxy group, especially

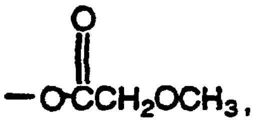, 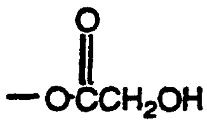 and 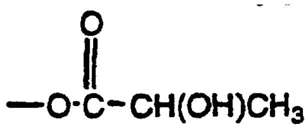.

Preferred alkenoate esters are the $C_{10}$-$C_{20}$ alkenoates and include $C_{14}$ to $C_{18}$ alkenoates, such as cis-7-hexadecenoate.

The term "amino acid ester" as used herein includes α-aminoalkanoyloxy, natural i.e., (L)-α -amino acid ester groups, e.g. the ester of glycine, peptide esters thereof, unnatural α-amino acid ester groups such as O-CO-CH($NH_2$)($CH_2$)$_3$ $CO_2H$, OCOCH($NH_2$)(CH)$_2$$NH_2$,OCOCH($NH_2$)(CH)$_3$$NH_2$

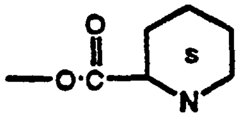

and α-amino alkanoates represented by the formula -OCOCH ($NR_{20}R_{21}$)$R_{22}$
wherein $R_{20}$ and $R_{21}$ are independently hydrogen or ($C_1$-$C_8$) straight or branched chain alkyl groups or $R_{20}$ and $R_{21}$ together with N form a 4, 5 or 6 membered ring optionally substituted with $NR_{21}$, -O- or -S- and $R_{22}$ is H, $CH_3$, $CH_2OH$, $CH(OH)CH_3$, $CH_2SH$,

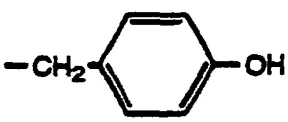, 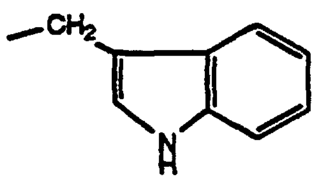

$CH_2CONH_2$, -$(CH_2)_2CONH_2$, $CH(CH_3)_2$, $CH(CH_3)_2$, $CH(CH_3)C_2H_5$, $(CH_2)_2SCH_3$, $CH_2$,$CO_2H$, $(CH_2)_2CO_2H$, $(CH_2)_4NH_2$, -$CH_2C_6H_5$,

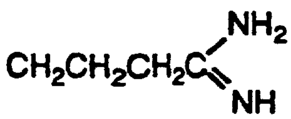,

and pharmaceutically acceptable acid addition salts thereof, or ($C_1$-$C_8$) straight and branched chain alkyl groups optionally substituted by hydroxyl or $NR_{20}R_{21}$. Preferred amino acid acids are the natural α amino acid groups, dipeptides and α -amino alkanoates wherein $R_{20}$ and $R_{21}$ are each $CH_3$. The most preferred amino acid esters are those derived from alanine, phenylanine, glycine, leucine, isoleucine and valine.

The term "carboxylic acid ester" as used herein means those acid esters represented by the formula

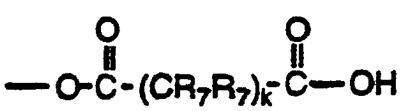

wherein each $R_7$ is as defined herein above, and where more than one $R_7$ appears in a molecule, each $R_7$ may be the same or different from the others, and k is an integer of from 1 to 8. Typically suitable acid esters include oxalic, malonic, succinic, glutaric and adipic acids as well as branched chain diacids such as

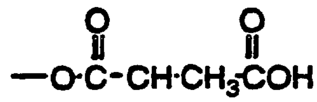

Preferred salts are HCl and lactic acid.

The term "carbonic and carbamic acid esters" means

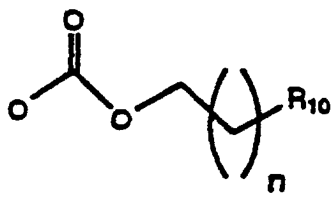 and 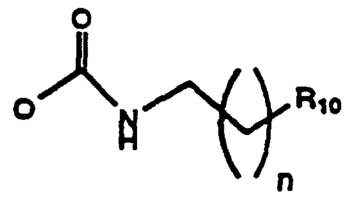

respectively,

wherein n is 1, 2 or 3;

$R_{10}$ is OH, NHR or a salt thereof; and

R is H, $CH_3$ or $C_2H_5$;

The term "LG" means a leaving group, and unless otherwise noted may be Cl, Br, I, $OSO_2CH_3$, $OSO_2C_6H_5$, $OSO_2(C_6H_4)CH_3$, $OSO_2(C_6H_4)Cl$, $OSO_2(C_6H_4)Br$, $OSO_2(C_6H_4)NO_2$, $OSO_2(CF_2)_pF$ where p is 1 to 4.

The term "Hal" means halogens, and unless otherwise noted may be Cl Br and I.

The term "PG" means a protecting group, and unless otherwise noted may be $CH_2Ph$ (benzyl), $CH_2OCH_3$ (MOM), $CH_2OCH_2Ph$ (BOM), $CH_2OCH_2CH_2OCH_3$ (MEM), $CH_2OCH_2CH_2Si(CH_3)_3$ (SEM)

Compounds of the invention exhibited the following <u>in</u> <u>vitro</u> antifungal activity in EMEM against 11 strains of *C. albicans* and *tropicalis:* geometric mean MICs in the range of ≥0.01 to ≥0.10 (mcg/ml).

The most preferred compound of the invention.

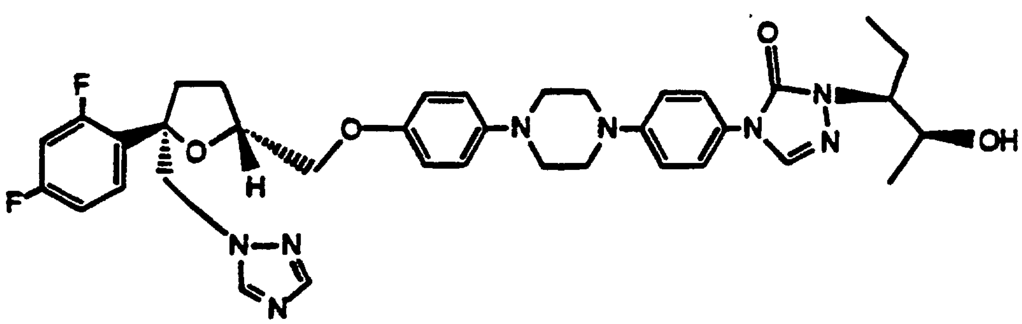,

exhibited the following in vitro antifungal activity in EMEM against 11 strains of *C. albicans* and *tropicals*: geometric mean MICs of ≥0.01 (mcg/ml).

Compounds of the invention also exhibited in vivo antifungal activity.

The more preferred esters listed hereinabove are water soluble and readily convertible in vivo to the corresponding alcohols e.g. $R_5$ is

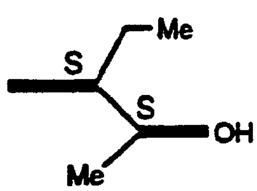 or 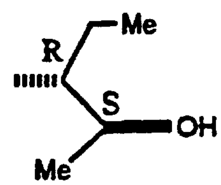

Compounds of the present invention as illustrated by reference to formula Ia below can exist in the "cis " isomeric form. With reference to formula Ia, for example,

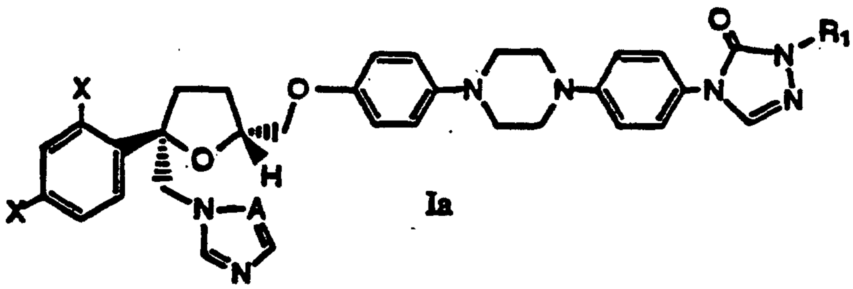

Ia

"cis" means that the

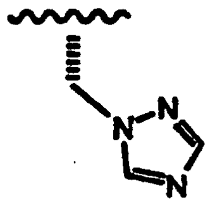

moiety and the

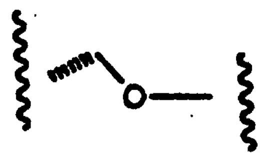

moiety are on the same side of the of the tetrahydrofuran ring.
The same meaning for the term "cis" attaches to formulas, IIIa, IVa of the present invention and the structures of the final products of the invention.

By relative stereochemistry, it is understood that the compound just below:

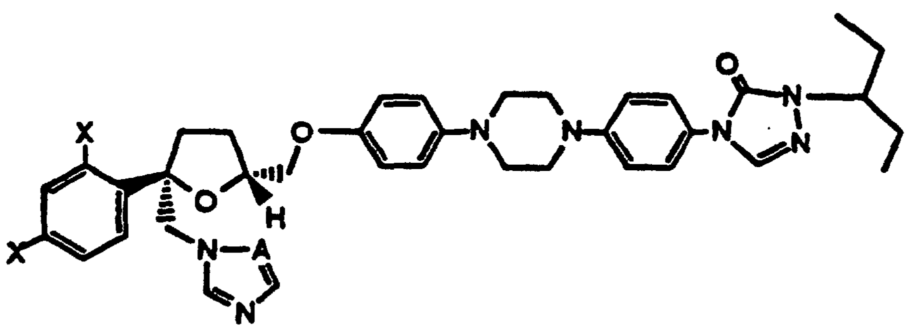

can either have the stereochemistry shown or be the enantiomer thereof, that is

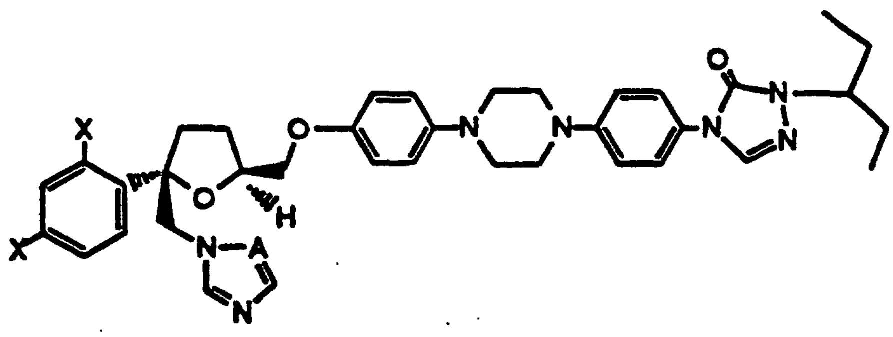

Unless otherwise indicated, structures of other compounds throughout this application which show the cis stereochemistry about the tetrahydrofuran ring are to be understood as encompassing the compound shown or the enantiomer thereof.

Ring numbering for certain positions on the tetrahydrofuran ring is illustrated by reference to formulas Ia and Ib just below.

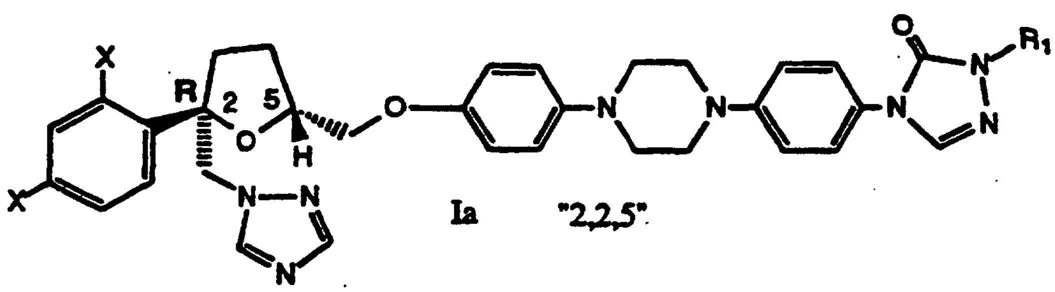

Ia "2,2,5"

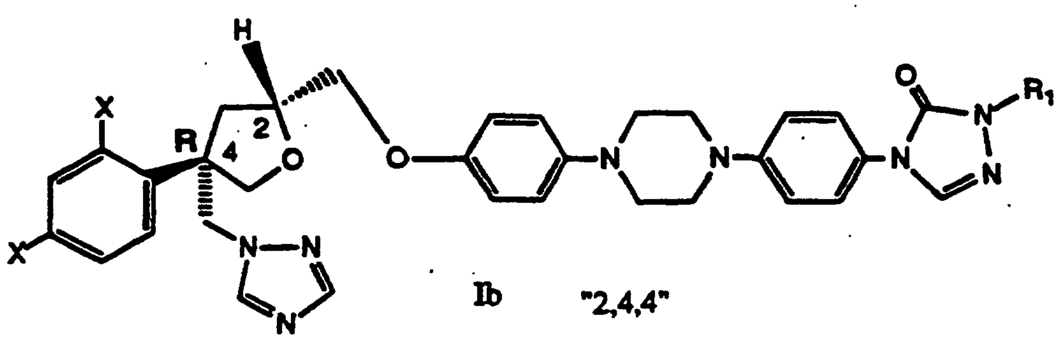

Ib "2,4,4"

From the formulas just above, it can be seen that formula Ia compounds of the invention are known as 2,2,5 compounds, that formula Ib compounds of the invention are known as 2,4,4 compounds.

GENERAL SYNTHETIC PREPARATIONS

Compounds of the invention may be prepared according to the reaction schemes shown just below. It will be appreciated that in the following examples those marked with a [*] are included for illustrative purposes and that analogous schemes may be used to provide compounds according to the present invention. Preferred schemes are shown from Scheme 3 onward. The starting materials in these reaction schemes are either known or can be prepared according to known methods.

## SCHEME 1: 2,4,4-Tetrahydrofuran Compounds,

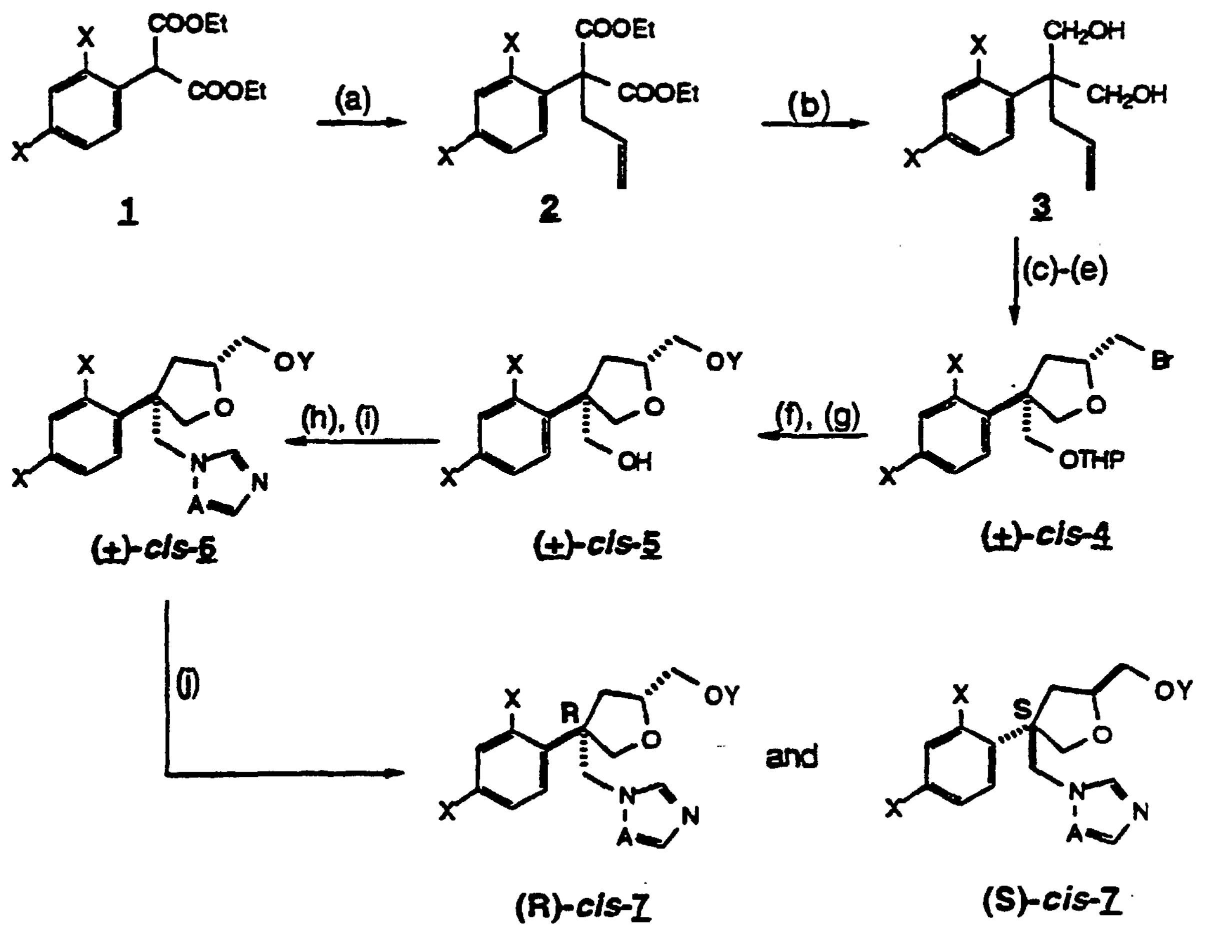


1 2 3

(±)-cis-6 (±)-cis-5 (±)-cis-4

(R)-cis-7 and (S)-cis-7

**Reagents:** (a) NaOEt, EtOH, allyl bromide ; (b) $LiAlH_4$, EtOEt ; (c) $Br_2$, $CH_2Cl_2$ ; (d) chromatography ; (e) Dihydropyran, $H^+$ ; (f) $YO^-$ ; (g) $H^+$ ; (h) Mesylation ; (i) Azole anion (Z=N,CH), DMF, heat ; (j) Chiralcell chromatography, using a Daicel OD, Chiralcell column.

The reactions shown in the scheme 1 just above can be carried out in a manner analogous to that set forth in U. S. Patent 5,039,676, column 23 , line 42 through column 24, line 7. U. S. Patent 5,039,676 is hereby incorporated by reference.

**SCHEME 2: 2,4,4-Tetrahydrofuran Compounds,**

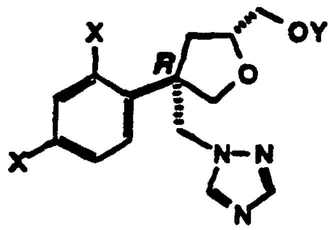

X = Cl or F;

Y = 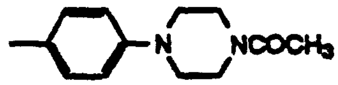

(R)-*cis*-7

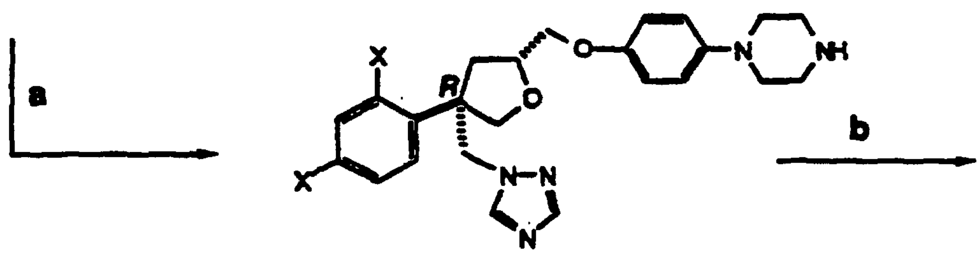


8

**SCHEME 2: contd.**

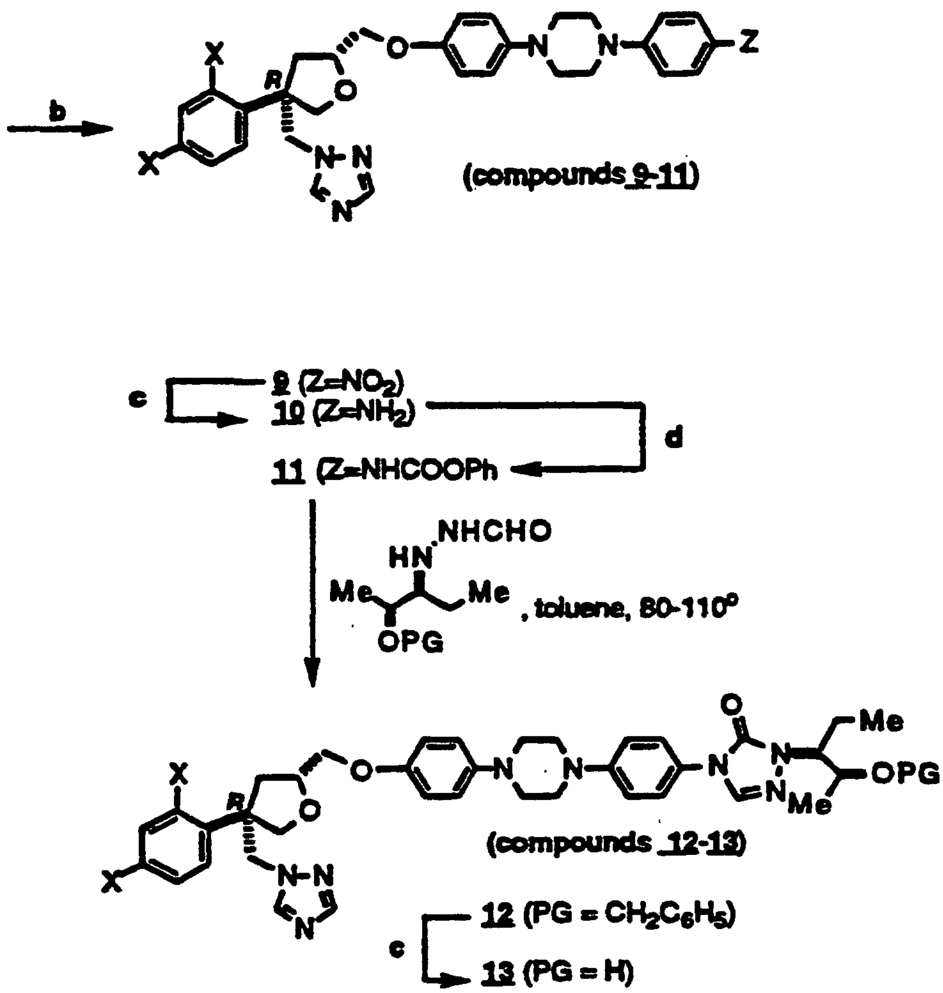


**Reagents:** (a) NaOH, n-BuOH; (b) p-Cl-$C_6H_4NO_2$, $K_2CO_3$, DMSO; (c) $H_2$, Pt-C; (d) $C_6H_5OCOCl$, pyridine, $CH_2Cl_2$.

The reactions shown in the scheme 2 just above can be carried out in a manner analogous to that set forth in scheme V, page 28 and scheme VI, page 29 of commonly assigned copending U. S. 5 661 151, filed June 2, 1995. Compounds of formula 14 just below:

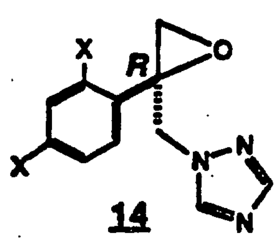

14

can be prepared in a manner analagous to that set forth in scheme VI, page 28 of commonly assigned copending US

5 661 151, filed June 2, 1995.

**Scheme 3**

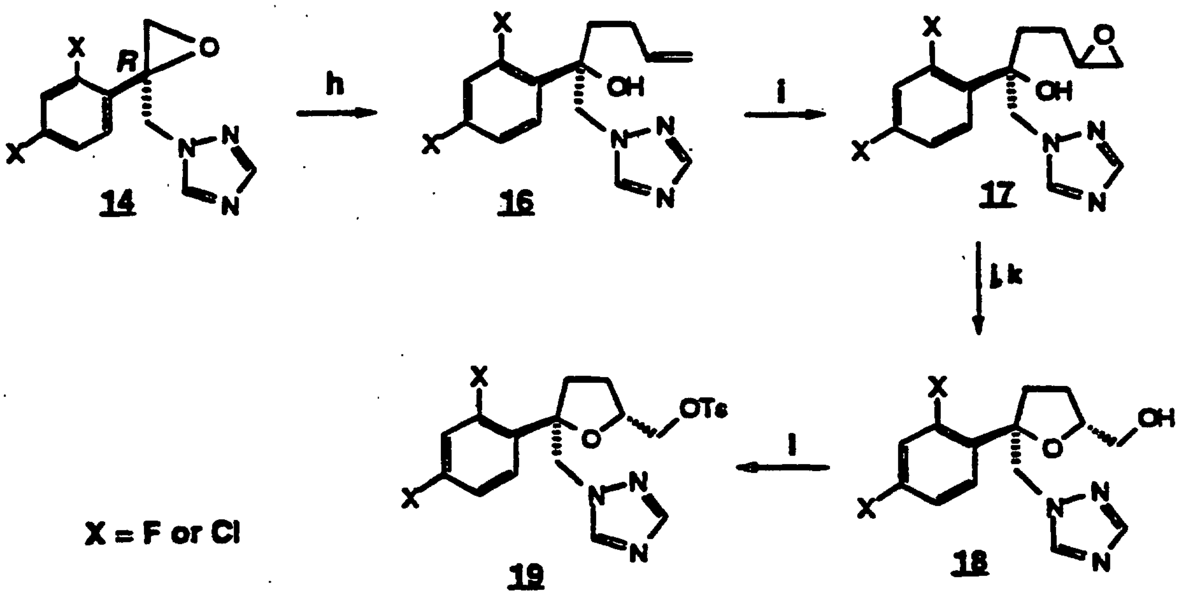


**Reagents**: (h) allyl magnesium bromide, CuI, THF; (i) *m*-chloroperbenzoic acid; (j) $Et_3N$, toluene, Δ; (k)chromatography; (l) tosyl chloride, $Et_3N$.

Compounds of formula **19** can be prepared as shown in scheme 3 above by treating a suitably substituted chiral epoxide, e.g **14**, with an allyl magnesium halide in an appropriate solvent, e.g. THF, in the presence of a catalyst, e.g. CuI, at a suitable temperature, e.g -70°C to 0°C, to produce an hydroxylic olefin, e.g. **16**. The hydroxylic olefin so produced can be treated with an appropriate oxidizing agent, e.g. *m*-chloroperbenzoic acid, in a suitable solvent, e.g. $CH_2Cl_2$, at 0°C to 25°C to produce an hydroxylic epoxide, e.g **17**. The hydroxylic epoxide so produced can be treated with a tertiary amine base, e.g. $Et_3N$, or NaH in a suitable inert solvent, e.g. toluene, at an elevated temperature, e.g. 90°C to 110°C, to produce an hydroxylic tetrahydrofuran, e.g. **18**. The hydroxylic tetrahydrofuran so produced can be treated with an alkyl- or arylsulfonyl halide, e.g. toluenesulfonyl chloride, in the presence of an organic amine base, e.g. $Et_3N$ or N,N-dimethylaminopyridine, in a suitable solvent to produce the corresponding sulfonate, e.g. **19**,

**Scheme 4**

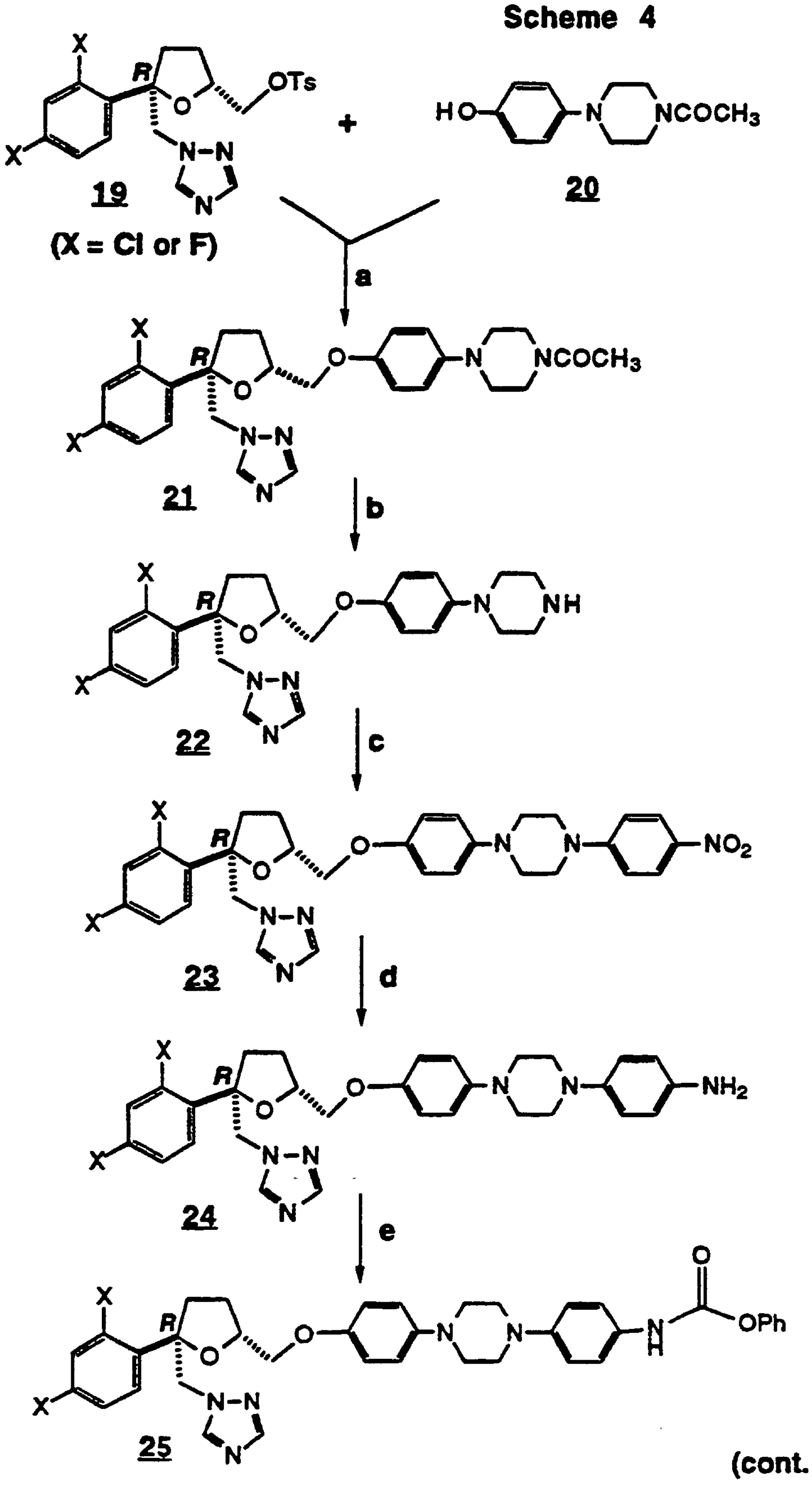


19 (X = Cl or F)

20

21

22

23

24

25

(cont. next page)

**(25)**

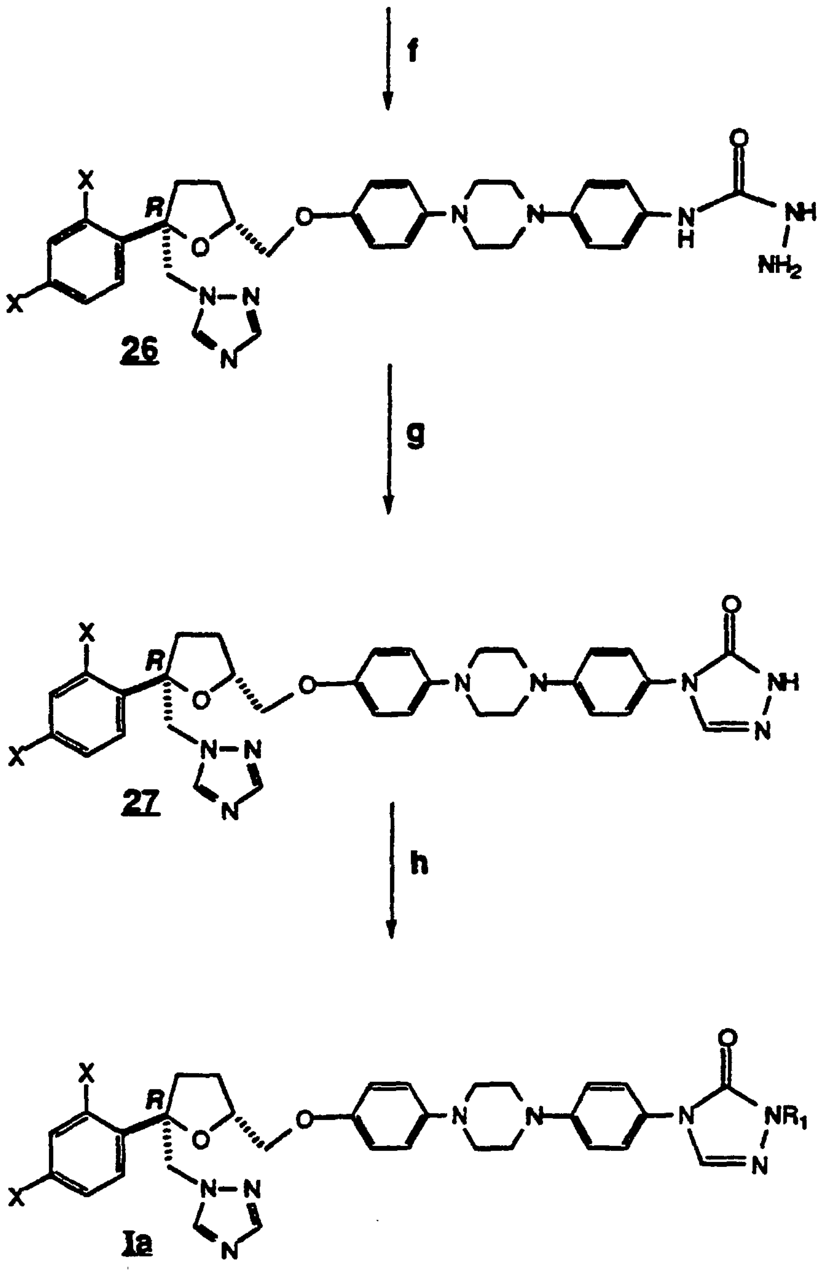


**26**

**27**

**Ia**

**Reagents:** (a) NaH, DMF; (b) NaOH, n-BuOH; (c) *p*-Cl-$C_6H_4NO_2$, $K_2CO_3$, DMSO; (d) $H_2$, Pt-C; (e) $C_6H_5OCOCl$, pyridine, $CH_2Cl_2$; (f) $NH_2NH_2.H_2O$, $H_2O$, dioxane; (g) formamidine acetate, DMF, 80°C; (h) according to Scheme II, pg 25 and Examples 19 and 20, of U.S. Ser. No. 08/460,752, filed June 2, 1995

The compounds of formula Ia can be prepared from compounds of formula **19** by the reactions shown in scheme 4 above carried out in a manner analogous to that set forth in scheme II, page 24, of commonly assigned copending U.S. 5 661 151, filed June 2, 1995.

If, in the above reaction scheme 3, the S epoxide is used:

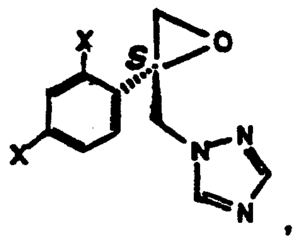

then the corresponding final product of different stereochemistry will be obtained from scheme 4. For example, using such a starting epoxide, the following product was obtained:

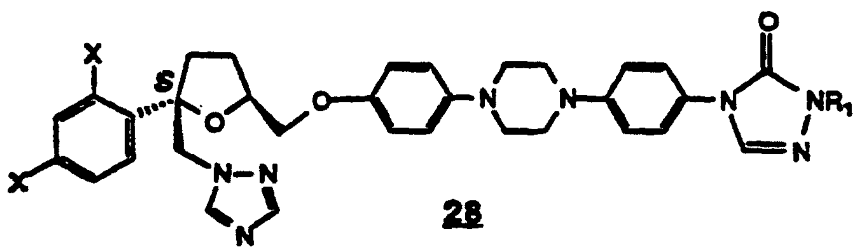

28

As can be seen from the above formulas and from other formulas in the specification, S or R is sometimes used to indicate the stereochemistry of a particular chiral center of a molecule.

SCHEME 5

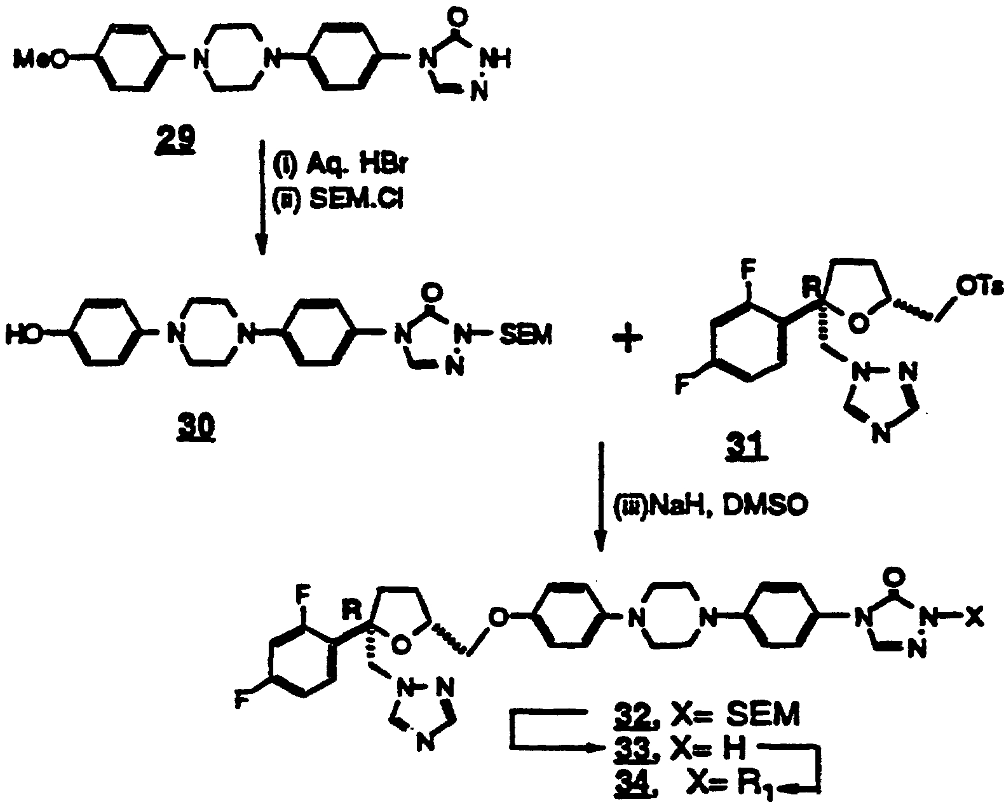


29

30

31

32, X= SEM
33, X= H
34, X= $R_1$

The reactions shown in the scheme 5 just above can be carried out in a manger analogous to that set forth in scheme 4, page 27 of commonly assigned copending U.S. 5 661 151, filed June 2, 1995. As used herein, SEM means $CH_2OCH_2CH_2SiMe_3$.

SCHEME 6

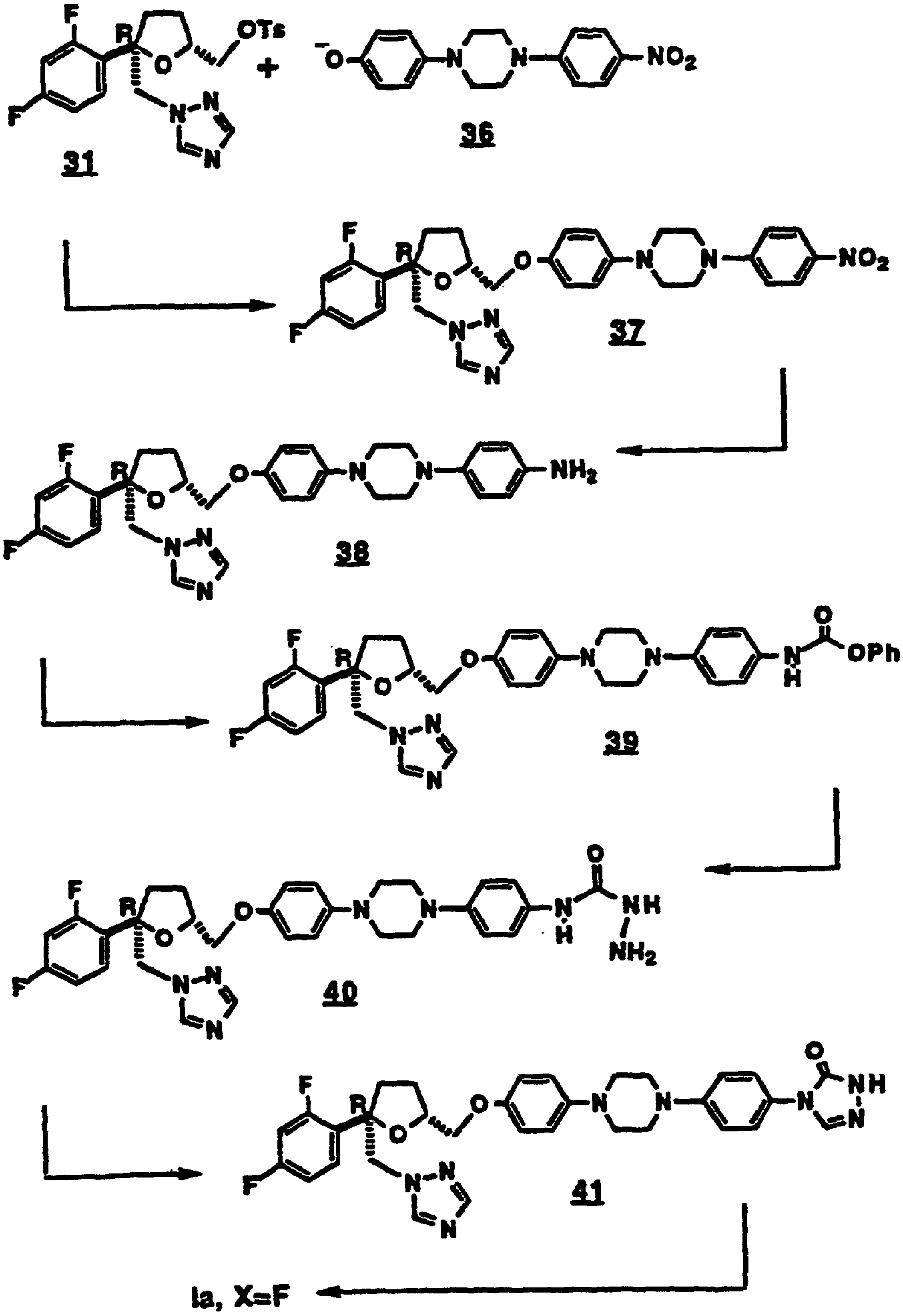

31

36

37

38

39

40

41

Ia, X=F

The reactions shown in the scheme 6 just above can be carried out in a manner analogous to that set forth in scheme V, page 28 of commonly assianed copending U.S. 5661151. filed June 2. 1995.

**Scheme 7**

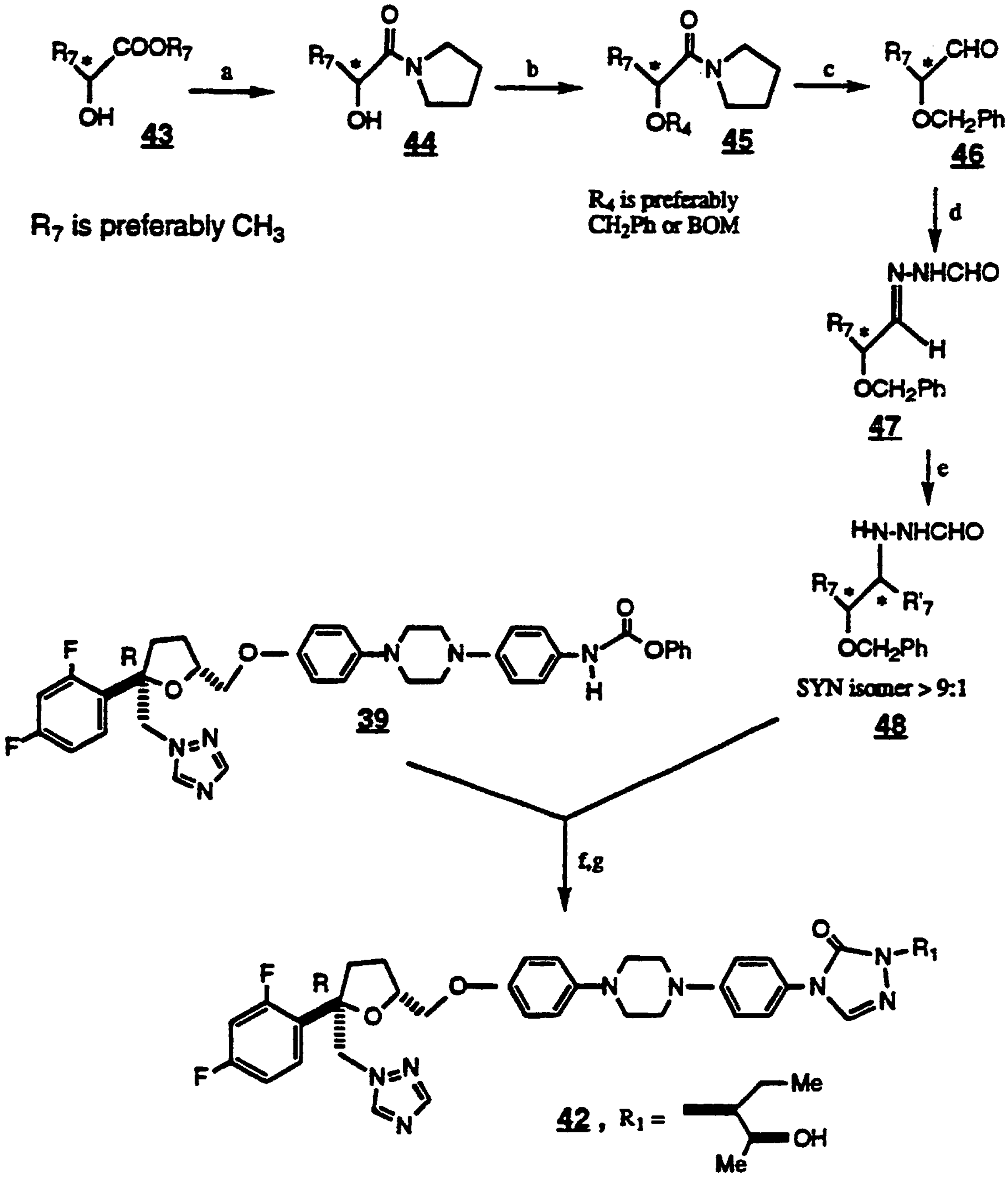


43, 44, 45, 46, 47, 48, 39, 42

(a) pyrrolidine, r.t., 24 h; (b) $R_7$-X, NaH, DMF; (c) RED-AL, toluene. -20°; (d) $H_2NNHCHO$, MeOH; (e) $R'_7MgBr$, $Et_2O$, -10°C to r.t., 24 h; (f) toluene, DBU, 80°C, 6 Hours; 100°C, 16 hours; (g) $H_2$, Pd, HCOOH, 80°C.

wherein $R_4$ is $CH_2Ph$, $CH_2CH_3$ (MOM), $CH_2OCH_2CH_2OCH_3$ (MEM), $CH_2OCH_2CH_2SiMe_3$ (SEM), or $CH_2OCH_2Ph$ (BOM).

The reactions shown in the scheme 7 just above can be carried out in a manner analogous to that set forth in scheme VI, page 29 of commonly assigned copending U.S. 5 661 151, filed June 2, 1995. LG is a leaving group such as chlorine.

**Scheme 8**

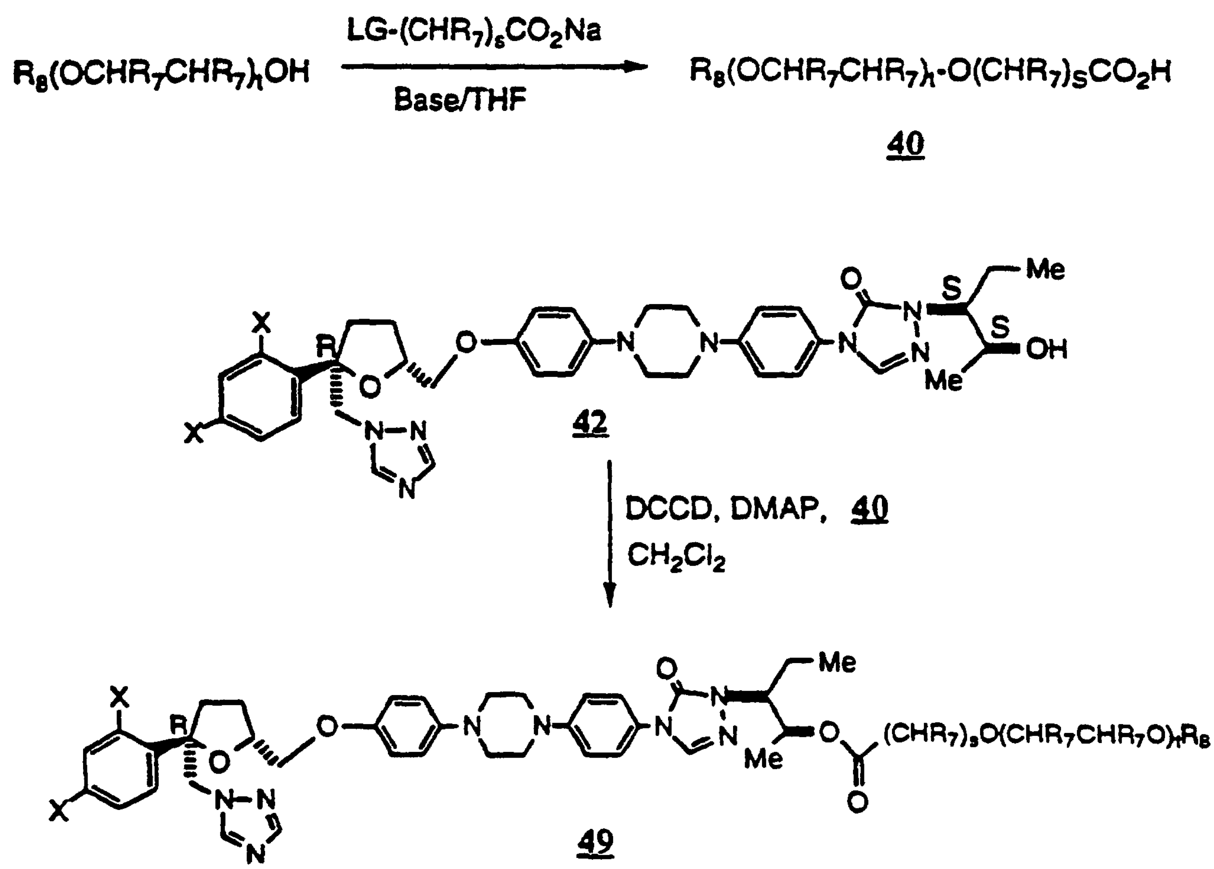


The reactions shown in the scheme 8 just above can be carried out in a manner analogous to that set forth in scheme VII, page 30 of commonly assigned copending U.S. 5 661 151, filed June 2, 1995.

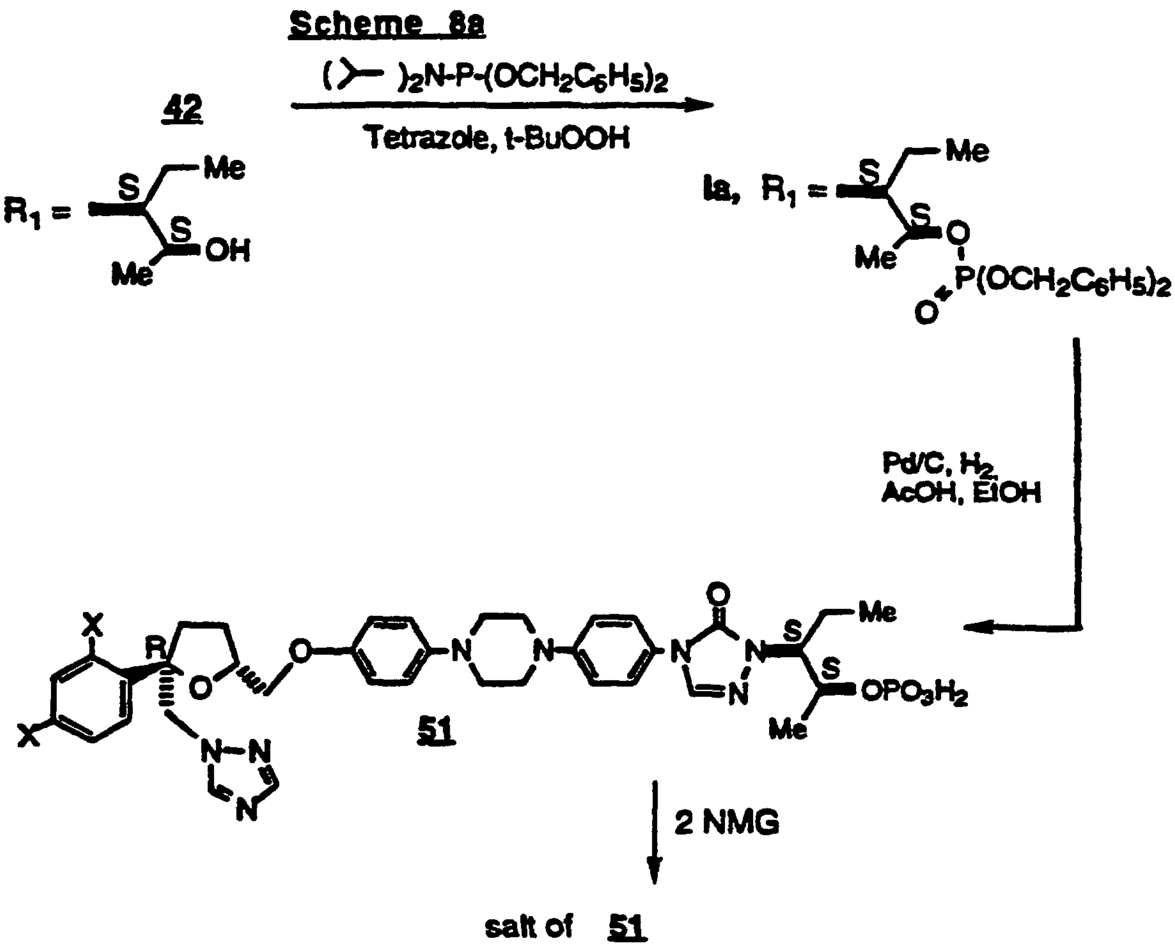


The reactions shown in the scheme 8a just above can be carried out in a manner analogous to that set forth in scheme VIIIA, page 31 of commonly assigned copending U.S. 5 661 151, filed June 2, 1995.

Scheme 9

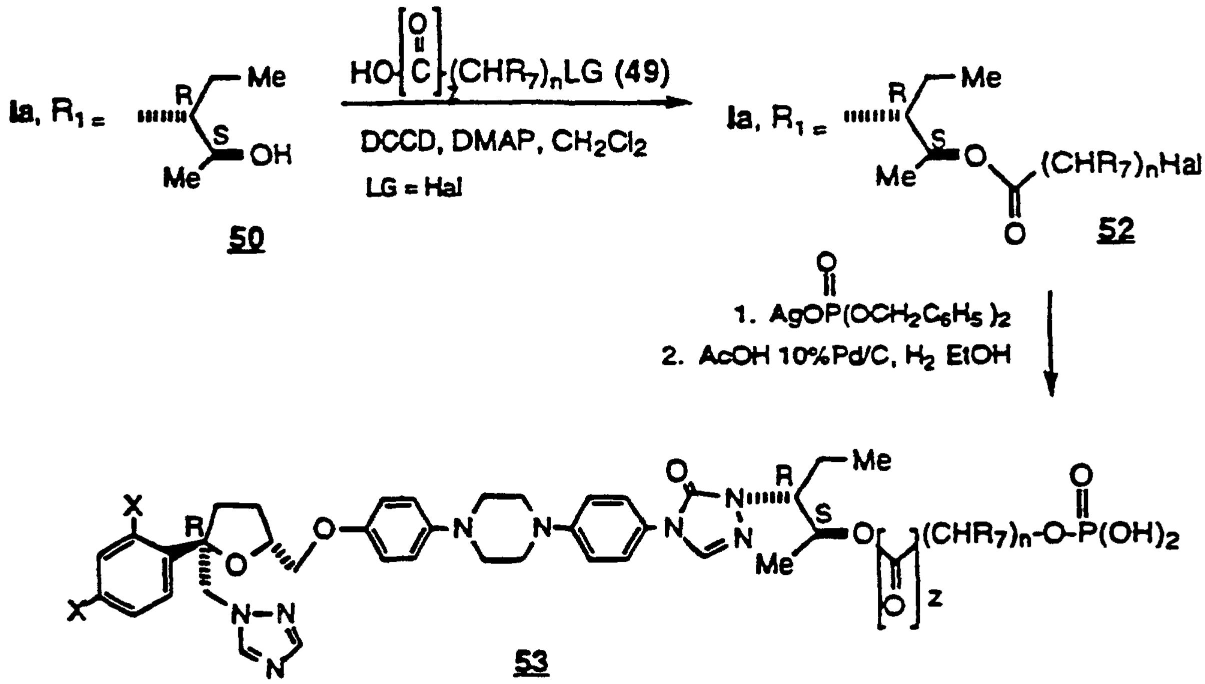


50

52

53

Hal=Br or Cl.

The reactions shown in scheme 9, above can be carried out in a manner analogous to that set forth in VIIIB, page 32 of commonly assigned copending U.S. 5 661 151, filed June 2, 1995.

In the table just above, the compound Ia, when $R_1$ is

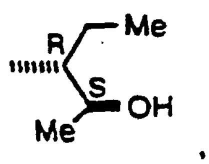

is also referred to as compound 50. Similarly, two labels are also used for compound 52

## Scheme 10

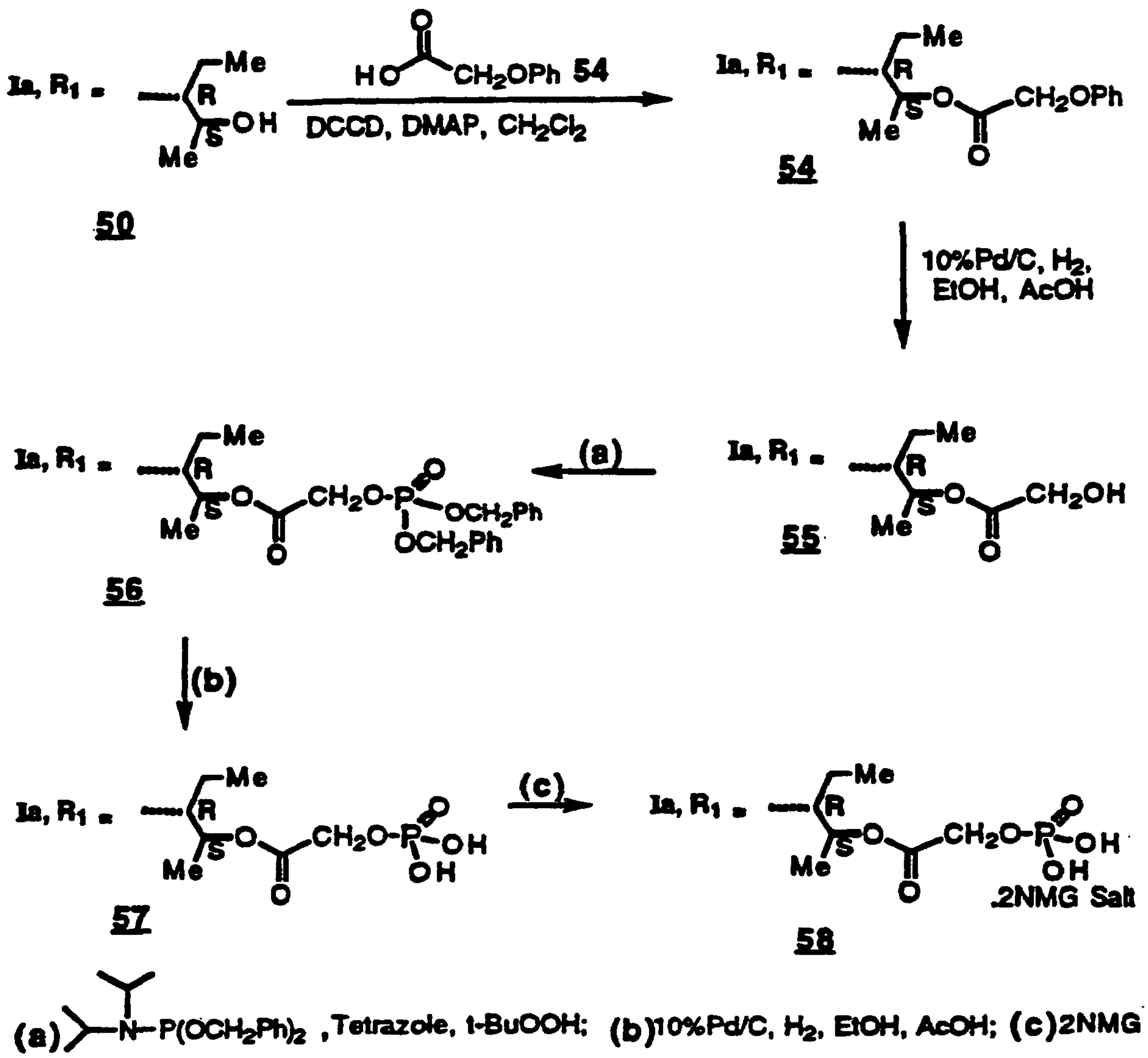


The reactions shown in scheme 10 just above can be carried out in a manner analogous to that set forth in scheme VIIIC, of page 34 of commonly assigned copending U.S. Ser. No. 5 661 151, filed June 2, 1995.

**Scheme 11**

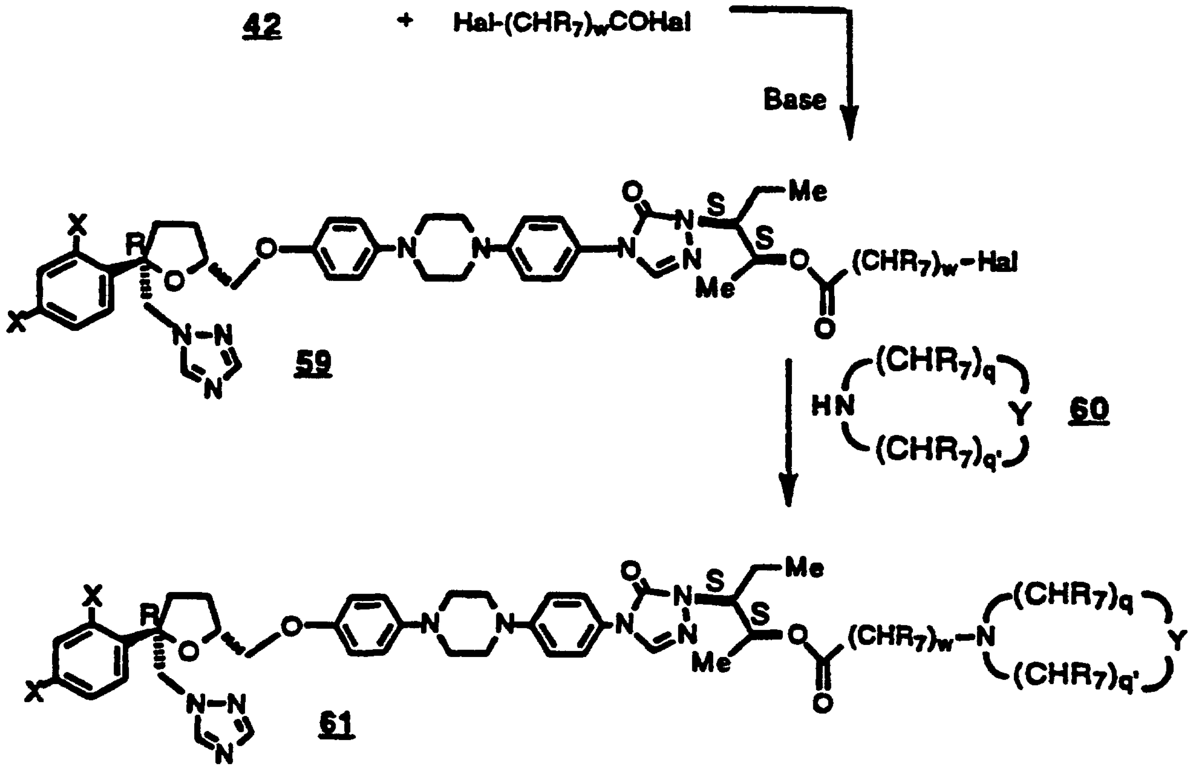


The reactions shown in the scheme 11, just above can be carried out in a manner analogous to that set forth in scheme IX, page 36 of commonly assigned copending U.S. 5 661 151, filed June 2, 1995.

**Scheme 12**

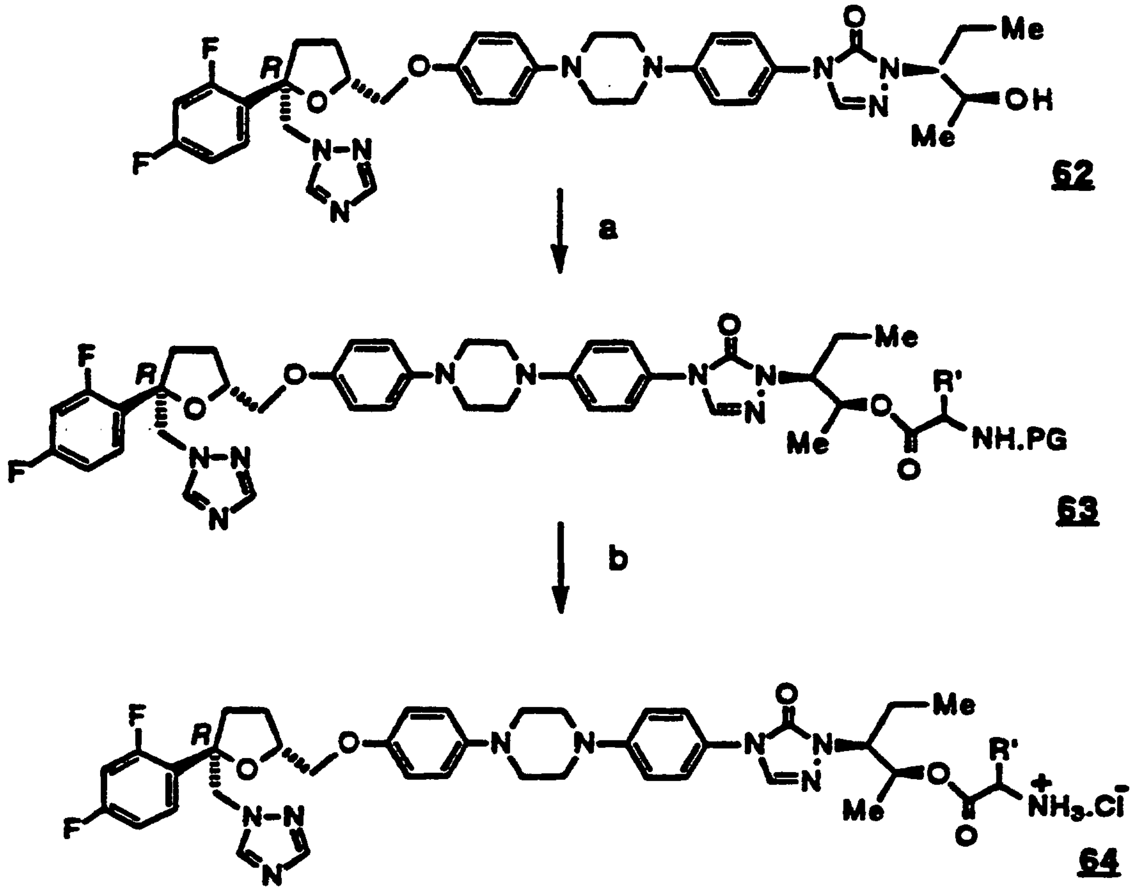


62

63

64

**Reagents: (a)** 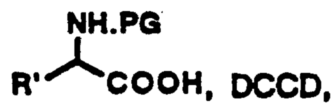**, DCCD, DMAP, $CH_2Cl_2$; (b) HCl in dioxane**

Where R'= H, $CH_3$, $CH_2OH$, $CH(OH)CH_3$, $CH_2SH$, $CH_2CONH_2$, $CH_2CH_2CONH_2$, $CH(CH_3)_2$, $CH(CH_3)CH_2CH_3$, $CH_2CH_2SMe$, $CH_2COO^-$, $CH_2CH_2COO^-$, $(CH_2)_4NH_3$,

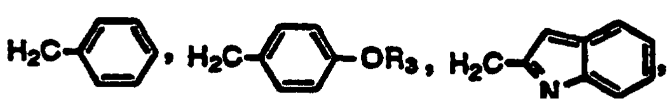

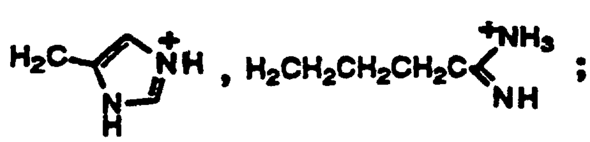

$R_3$= H, Phosphate ester, Sulphate ester,
PG=$CH_2Ph$

The reactions shown in the scheme 12, just above can be carried out in a manner analogous to that set forth in scheme X, page 37 of commonly assigned copending U.S. 5 661 151, filed June 2, 1995.

Using an appropriate amino acid and an appropriate compound in place of compound 62, the reaction scheme 12, shown just above, can be carried out by one skilled in the art so as to arrive at any amino acid ester salts of the

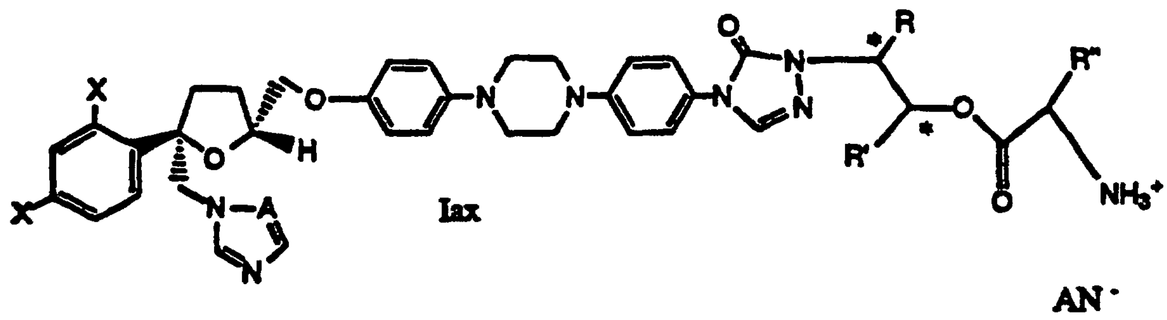

Iax

and

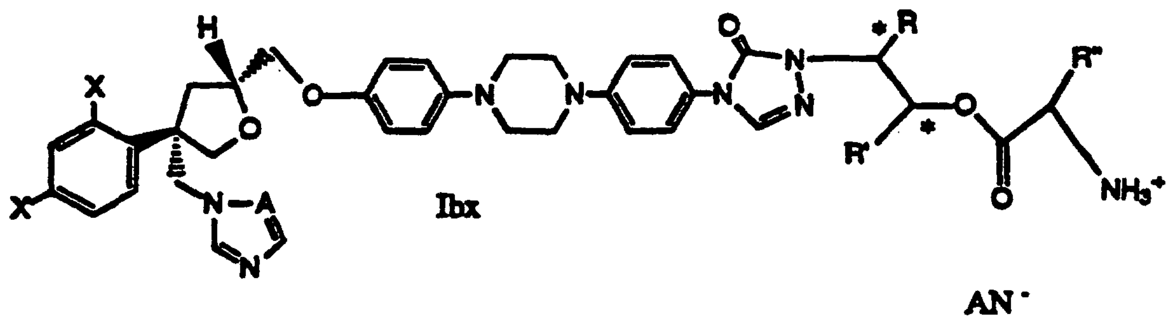

Ibx

Scheme 13

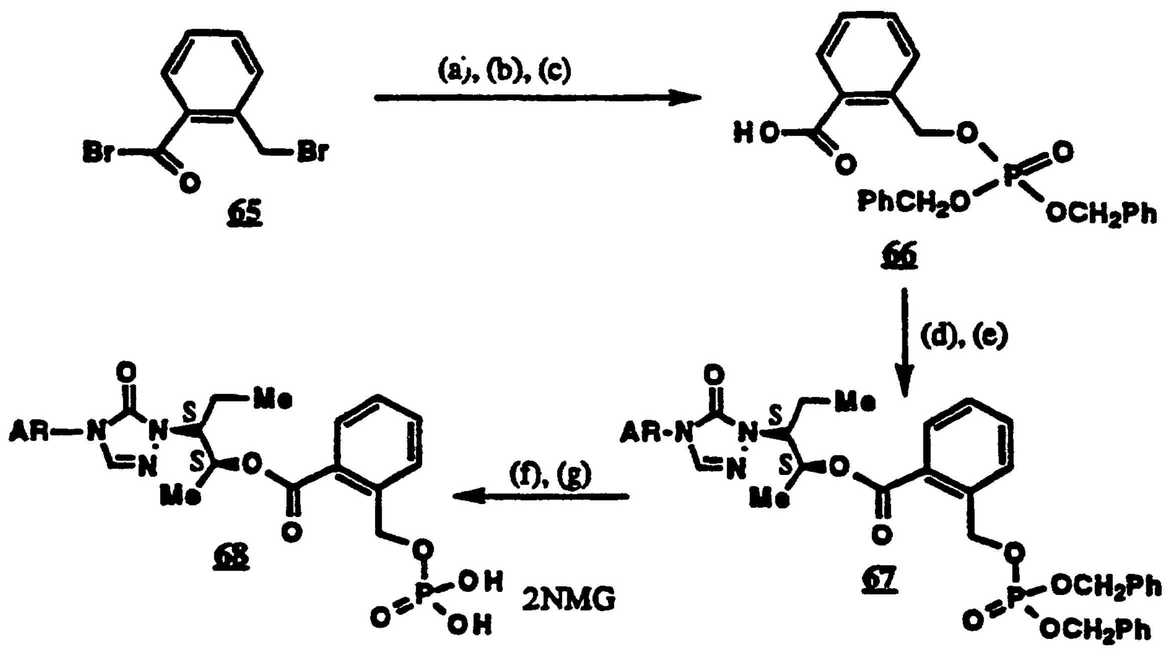


65 66 67 68

Reagents: (a) 2,2,2-trichloroethanol; (b) silver dibenzyl phosphate; (c) Zn, HOAc-THF; (d) $SOCl_2$; (e) 42; (f) $H_2$, 10% Pd-C; (g) 2eq. N- methyl glucamine.

As used in the Schemes 13 and 14 AR is

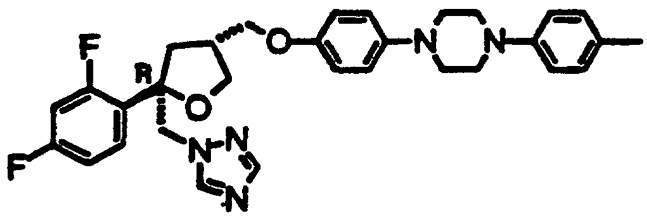

The reactions shown in the scheme 13, just above can be carried out in a manner analogous to that set forth in scheme XIA, page 38 of commonly assigned copending U.S. 5 661 151, filed June 2, 1995.

Scheme 14

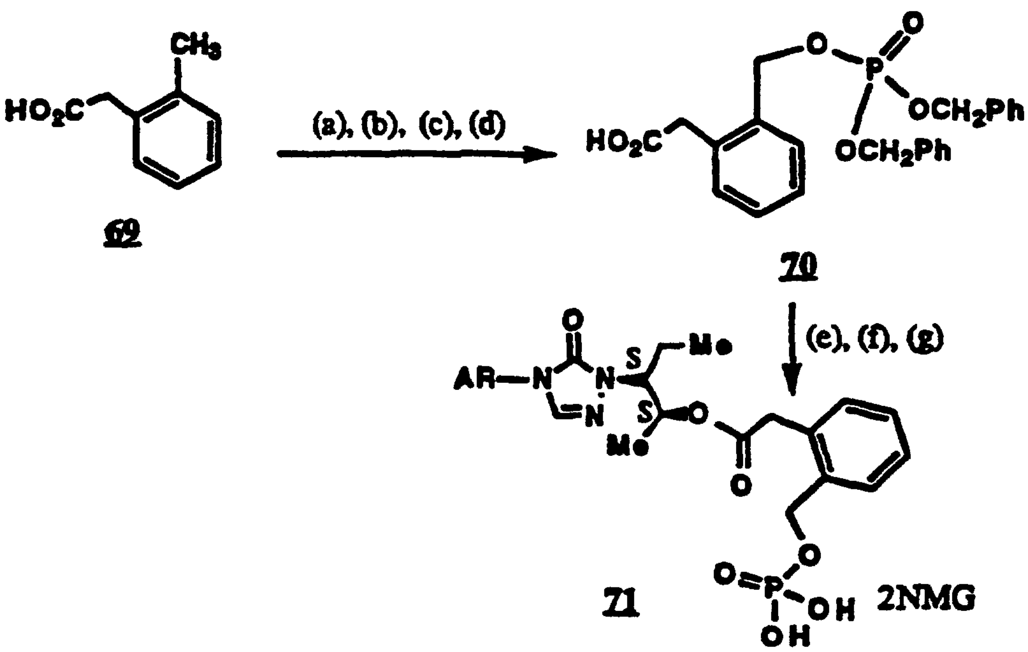


69

70

71

Reagents: (a) 2,2,2-trichloroethanol, DCCD, DMAP; (b) N-bromosuccinimide;

(c) silver dibenzyl phosphate; (d) Zn, HOAc-THF; (e) compound 42

DCCD, DMAP; (f), $H_2$, 10% Pd-C; (g) 2eq. N- methyl glucamine

The reactions shown in the scheme 14, just above can be carried out in a manner analogous to that set forth in scheme XIB, page 39 of commonly assigned copending U.S. 5 661 151, filed June 2, 1995.

The compounds of this invention are prepared in accordance with Schemes hereinabove and the following Examples using commercially available starting materials, or using starting materials which can be prepared by known methods.

<u>EXAMPLE OF THE PREPARATION OF</u> [(R)-CIS]-4-[4-[4-[4-[[5-(2,4-DIFLUOROPHENYL)-TETRAHYDRO-5-[(1H-1,2,4-TRIAZOL-1-YL)METHYL]-2-FURANYL]METHOXY]PHENYL]-1-PIPERAZINYL]PHENYL]-2-(1(S)-ETHYL-2(S)-HYDROXYPROPYL)-2,4-DIHYDRO-3H-1,2,4-TRIAZOL-3-ONE (**<u>42</u>**)

which has the structural formula

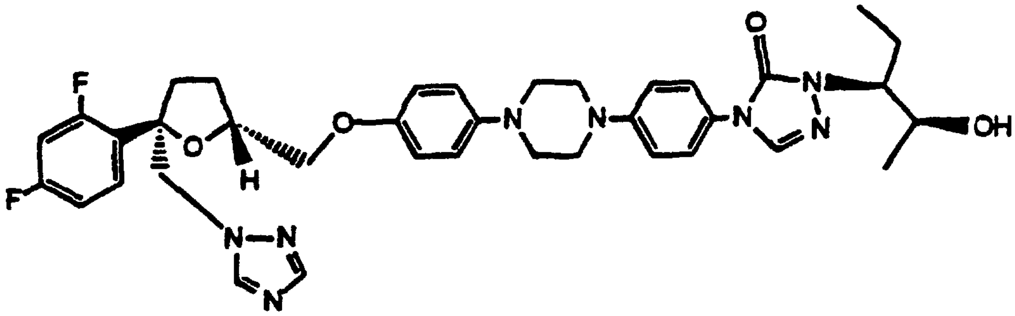

COMPOUND **16, X=F**

Add copper(I) iodide (50 mg) to a solution of **15, X=F** (16 g) in anhydrous THF (300 mL). Stir the mixture and cool it to -70°C, and then slowly add a solution of 2.0 M allylmagnesium chloride in THF (52 mL). Allow the mixture to warm to r.t. and stir for an additional 3 hr. Quench the reaction with 1:1 saturated brine-10% aq. $KH_2PO_4$, extract with EtOAc, dry the extract over anhydrous $Na_2SO_4$, and filter the mixture. Evaporate the solvent and chromatograph the residue on silica gel with acetone-hexane to obtain the title compound **16, X=F** (9.7 g), $M^+$ 280.3.

COMPOUND **17, X=F**

A solution of the above olefin **16, X=F** (11.2 g) in $CH_2Cl_2$ (250 mL) at -10°C is slowly treated with 85% purity *m*-chloroperbenzoic acid (9.0 g), and the mixture is then stirred at r.t. for 24 hr. The reaction mixture is washed with cold 5% aq. NaOH, then water, then saturated brine. The solution is dried over anhydrous $MgSO_4$, filtered, and the filtrate evaporated to leave the title compound **17, X=F** (11.8 g), which is used without further purification.

COMPOUND **18, X=F**

Stir and heat at 80°C a solution of the epoxide **17, X=F** from above (10 g), toluene (125 mL), and $Et_3N$ (6.0 mL) for 2 hr. Suction-filter the hot mixture through a pad of silica gel and wash the pad with EtOAc. Evaporate the combined organics to leave a residue of a mixture of *cis-* and *trans-* alcohols. The residue is chromatographed on silica gel with MeOH-$CH_2Cl_2$ to give the title *cis*-compound **18, X=F** as the first of the isomers to elute.

COMPOUND **31** (**19, X=F**)

Stir a solution of the alcohol from above (12.4 g), $Et_3N$ (5.9 mL), and DMAP (0.5 g) in $CH_2Cl_2$ (200 mL). Add tosyl chloride (8.2 g) and stir for 4 hours. Wash the mixture with cold aqueous 5% $KH_2PO_4$, and dry the organic layer over anhydrous $Na_2SO_4$. Filter the mixture, evaporate the filtrate to a residue, and crystallize the residue from EtOAc-(i-Pr)$_2$O to obtain the tosylate **31** (4.2 g), mp 150-151°C.

COMPOUND **32**

Stir a mixture of compound **30** (1.5 g), scheme 5, and $Cs_2CO_3$ (1.25 g) in DMF (50 mL) for 0.75 hours. Add the tosylate **31** from above (1.4 g) and stir at 80°C for 18 hours. Pour the mixture into aqueous 5% $KH_2PO_4$ and extract with EtOAc. Evaporate the extract and chromatograph the residue on silica gel to obtain compound **32** (1.4 g).

COMPOUND **33**

Stir a mixture of the compound **32** from above (1.4 g) in 1,4-dioxane (8 mL) and 12N HCl (15mL) at 90°C for 18 hours. Pour the mixture in water, add $NaHCO_3$ until the pH=4, and extract with EtOAc. Evaporate the extract and boil the residue in CH3CN. Allow to cool and filter to leave compound **33** (0.7 g), FAB mass spectrum: $M^+$ 615.

COMPOUND **34,** $R_1$ = (S,S)-$CH(CH_3CH_2)[(CH(OCH_2OCH_2Ph)CH_3]$

Stir a mixture of compound **33** (0.7 g) and $Cs_2CO_3$ (0.4 g) in DMF (20 mL) for 0.75 hours.
Add compound **80** (0.8 g),

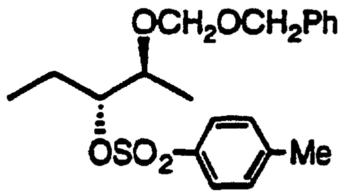

**80**

a compound which can be prepared by known means and stir at 80°C for 18 hours. Pour the mixture into aqueous 5% $KH_2PO_4$, extract with EtOAc, evaporate the extract, and chromatograph the residue on silica gel to obtain compound **34** wherein $R_1$ = (S,S)-$CH(CH_3CH_2)[(CH(OCH_2OCH_2Ph)CH_3]$ (0.5 g), FAB mass spectrum: $M^+$ 821.

COMPOUND **42**

Stir a solution of the compound from above (0.5 g) in 1:1 MeOH-6N HCl (20 mL) for 5 hours. Add $NaHCO_3$ until the pH>4. Extract with EtOAc, evaporate the extract, boil the residue in $Et_2O$, and filter to leave compound **42** (0.35 g), mp 165-167°C; $[\alpha]_D$ +5.5° (c=0.5, $CH_2Cl_2$).

The mass spectral data presented herein as $M^+$ are parent ions which were determined by Fast Atom Bombardment (FAB) technique and represent the $[M+H^+]$, i.e. {molecular ion+1} peaks.

Schemes 1-7 set forth the preferred processes to prepare the alcohol compounds of this invention.

As used in scheme 8 and elsewhere in this specification, the base 4-(N,N-dimethylamino)pyridine is referred to as "DMAP" and dicyclohexylcarbodiimide is referred to as DCCD.

The alkanoate and alkenoate esters of compounds of the invention are conveniently prepared by standard synthetic techniques, for example, by reaction of the anhydride or acid halide of the alkanoic acid or alkenoic acid in the presence of base e.g, pyridine.

The carbonic and carbamic esters of the compounds of the invention are conveniently prepared by standard synthetic techniques, for example, by the reaction of phosgene in the presence of a controlled amount of base, for example, pyridine, followed by reaction with an alcohol or amine in the presence of a base, for example, $Et_3N$ and DMAP.

The sulfate esters may be prepared by reaction of the alcohol compounds of formulas I to IV with sulfur trioxide in the presence of excess pryridine at temperatures of 70°-90°C for at least 2 hours in accordance with the procedure of R.M. Moriarty et. al. Tetrahedron Letters, Vol. 35, No. 44, p 8103-8106 (1994).

Compounds of the invention exhibit broad spectrum antifungal activity, in conventional antifungal screening tests, against human and animal pathogens, such as the following: *Aspergillus, Blastomyces, Candida, Cryptococcus, Coccidioides, Epidermophyton, Fonsecaea, Fusarium, Mucor, Saccharomyces, Torulopsis, Trichophyton, Trichosporon, Sporothrix and Pneumocysitis.*

The antifungal compounds of the invention and pharmaceutical compositons of this invention are expected to exhibit anti-allergic, antiinflammatory and immunomodulating activities, broad spectrum antiinfective activity, e.g., antibacterial, anti-protozoal and antihelminthic activities.

The present invention also provides a composition for treating or preventing fungal infections comprising an antifungally effective amount of a compound of the invention or a pharmaceutically acceptable salt thereof and a pharmaceutically acceptable carrier or diluent therefor.

The pharmaceutical compositions of the present invention may also contain a fungicidally effective amount of other antifungal compounds such as cell wall active compound. The term "cell wall active compound", as used herein, means any compound that interferes with the fungal cell wall and includes, but is not limited to, compounds such as papulacandins, echinocandins, and aculeacins as well as fungal cell wall inhibitors such as nikkomycins, e.g, nikkomycin K and others which are described in USP 5,006,513 which is hereby incorporated by reference.

The pharmaceutically acceptable salts of the compounds of the present invention include pharmaceutically acceptable acid and base addition salts.

The preferred pharmaceutically acceptable acid addition salts are nontoxic acid addition salts formed by adding to the compounds of the present invention about a calculated amount of a mineral acid, such as HCl, HBr, $H_2SO_4$, $HNO_3$ or $H_3PO_4$, or of an organic acid, such as an alkyl or arylsulfonic acid such as maleic, lactic, methanesulfonic, isithionic, para- toluenesulfonic, naphthylsulfonic and the like.

The pharmaceutically acceptable bases found suitable for use in the present invention are those which form pharmaceutically acceptable salts of the acidic pharmaceutically acceptable esters of the antifungal compounds of formulas, IIIa or IVa and include suitable organic and inorganic bases. Suitable organic bases include primary, secondary and tertiary alkyl amines, alkanolamines, aromatic amines, alkylaromatic amines and cyclic amines. Exemplary organic amines include the pharmaceutically acceptable bases selected form chloroprocaine, procaine, piperazine,

glucamine, N-methylglucamine, N-N-dimethyl glucamine ethylendediamine, diethanolamine, diisopropylamine, diethylamine, N-benzylenediamine, diethanolamine, diisopropylamine, diethylamine, N-benzyl-2-phenylethylamine, N-n'dibenzylethylenediamine, choline, triethylamine ("$ET_3N$"), tris(hydroxymethyl)aminomethane, or D-glucosamine. The preferred organic bases include N-methyl glucamine ("NMG"), diethanolamine, and tris(hydroxymethyl) aminomethane ("TRIS"). Use of two equivalents of NMG in this invention is more preferred. The suitable inorganic bases also include alkali metal hydroxides such as sodium hydroxide.

The pharmaceutical compositions of the present invention may be adapted for any mode of administration e. g., for oral, parenteral, e.g., SC, IM. IV and IP, topical or vaginal administration or by inhalation (orally or intranasally) Such compositions are formulated by combining the compound of the invention or an equivalent amount of a pharmaceutically acceptable salt of compound of the invention with an suitable, inert, pharmaceutically acceptable carrier or diluent.

Examples of suitable compositions include solid or liquid compositions for oral administration such as tablets, capsules, pills, powders, granules, solutions, suppositories, troches, lozenges, suspensions or emulsions. A solid carrier can be one or more substances which may also act as diluents, flavoring agents, solubilizers, lubricants, suspending agents, binders or tablet disintegrating agents; it can also be an encapsulating material. In powders, the carrier is a finely divided solid which is in admixture with the finely divided active compound. In the tablet, the active compound is mixed with carrier having the necessary binding properties in suitable proportions and compacted in the shape and size desired.

Topical dosage forms may be prepared according to procedures well known in the art, and may contain a variety of ingredients, excipients and additives. The formulations for topical use include ointments, creams, lotions, powders, aerosols, pessaries and sprays.

For preparing suppositories, a low melting wax such as a mixture of fatty acid glycerides or cocoa butter is first melted, and the active ingredients are dispersed homogeneously therein as by stirring. The molten homogeneous mixture is then poured into convenient sized molds, allowed to cool and thereby solidify.

Liquid form preparations include solutions, suspensions and emulsions. Liquid form preparations include, for example, water or water-propylene glycol solutions for parenteral injection. Liquid preparations can also be formulated in solution with an appropriate amount of a hydroxypropyl α- β or -γ-cyclodextrin having 2 to 11 hydroxypropyl groups per molecule of cyclodextrin, polyethylene glycol, e.g., PEG-200 or propylene glycol, which solutions may also contain water. Aqueous solutions suitable for oral use can be prepared by adding the active component in water and adding suitable colorants, flavors, stabilizing, sweetening, solubilizing and thickening agents as desired. Aqueous suspensions suitable for oral use can be made by dispersing the active component in finely divided form in water. A particularly preferred aqueous pharmaceutical composition may be prepared from the compounds of formulas I to IV together with hydroxypropyl-β-cyclodextrin in water. The use of derivatives of α-, β- and γ-cyclodextrins, for example, hydroxpropyl-β-cyclodextrin are disclosed by N. Bodor USP 4,983,586, Pitha USP 4,727,064 and Janssen Pharmaceutical International Patent Application No. PCT/EP 84/00417.

The pharmaceutical compositions of the present invention may be prepared by admixing the pharmaceutically acceptable carrier, e.g., a hydroxypropyl-β-cyclodextrin in water, and adding thereto an antifungally effective amount of a drug of the present invention. The solution so formed is filtered, and optionally, the water may be removed by well known methods, e.g., rotatory evaporation or lyophilization. The formation of the solution may take place at a temperature of about 15° to 35°C. The water is normally sterilized water and may also contain pharmaceutically acceptable salts and buffers, e.g., phosphate or citrate as well as preservatives. The molar ratio of the antifungal compound of formula I to hydroxpropyl-β-cyclodextrin is about 1:1 to 1:80, preferably 1:1 to 1:2. Normally the hydroxypropyl-β-cyclodextrin is present in molar excess.

Also included are solid form preparations which are intended to be converted, shortly before use, into liquid form preparations for either oral or parenteral administration. The solid form preparations intended to be converted to liquid form may contain, in addition, to the active materials, such as compounds of this invention, and optionally a cell wall active compound, especially a fungal cell wall inhibitor, e.g., a nikkomycin, flavorants, colorants, stabilizers, buffers, artificial and natural sweeteners, dispersants, thickeners, solubilizing agents and the like. The solvent utilized for preparing the liquid form preparations may be water, isotonic water, ethanol, glycerin, polyethylene glycols, propylene glycol, and the like, as well as mixtures thereof.

Parenteral forms to be injected intravenously, intramuscularly, or subcutaneously are usually in the form of a sterile solution, and may contain salts or glucose to make the solution isotonic.

The topical dosage for humans for antifungal use in the form of a pharmaceutical formulation comprising a compound of the invention (usually in the concentration in the range from about 0.1% to about 20% preferably from about 0.5% to about 10% by weight) together with a non-toxic, pharmaceutically acceptable topical carrier, is applied daily to the affected skin until the condition has improved.

In general, the oral dosage for humans for antifungal use ranges from about 1 mg per kilogram of body weight to about 30 mg per kilogram of body weight per day, in single or divided doses, with about 1 mg per kilogram of body

weight to about 20 mg per kilogram of body weight per day being preferred and the dose of about 1 mg per kilogram of body weight to about 10 mg per kilogram of body weight per day being most preferred.

In general, the parenteral dosage for humans for antifungal use ranges from about 0.25 mg per kilogram of body weight per day to about 20 mg kilogram of body weight per day, in single or divided doses, with about 0.5 to about 10 mg per kilogram of body weight per day being preferred.

The exact amount, frequency and period of administration of the compounds of the present invention for antifungal use will vary, of course, depending upon the sex, age and medical condition of the patent as well as the severity of the infection as determined by the attending clinician.

## Claims

1. A compound represented by a formula selected from the group consisting of

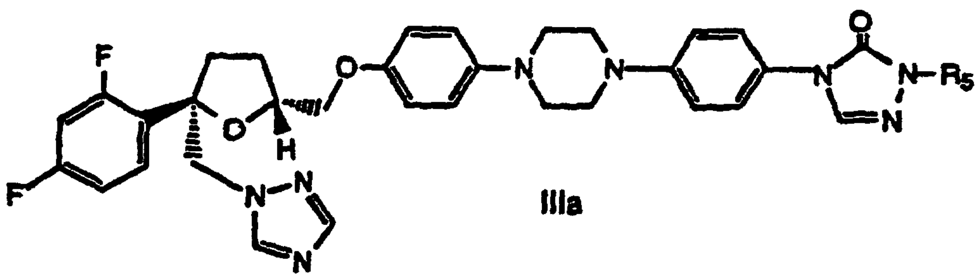

IIIa

wherein $R_5$ is

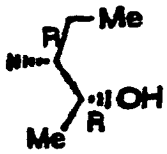 , 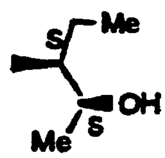 , 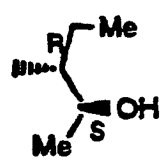 ,

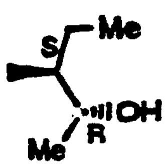 , 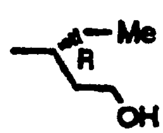 , 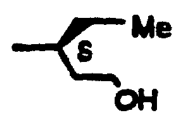 ,

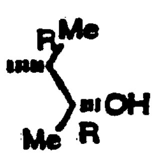 , 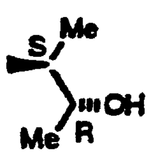 , 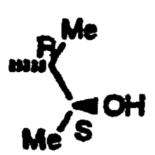 ,

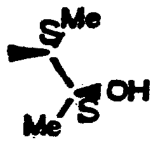 , 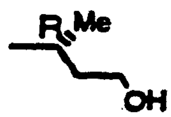 , 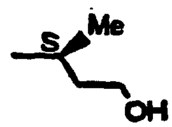 ,

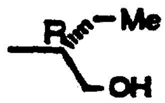 or 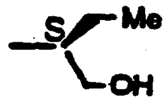

or an ester thereof or a pharmaceutically acceptable salt thereof, wherein the ester is a (a) polyether ester (b) phosphate ester (c) heterocyclic ester (d) alkanoate and alkenoate ester (e) amino-acid ester (f) carboxylic acid ester (g) carbonic and carbamic ester or (h) sulfate ester.

**2.** A compound selected from the group consisting of

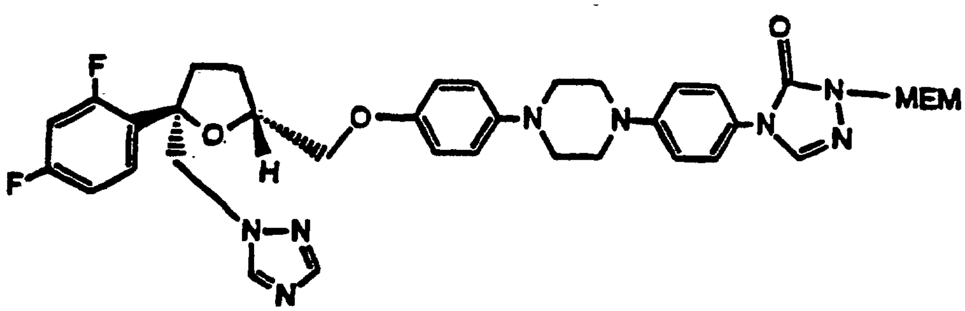

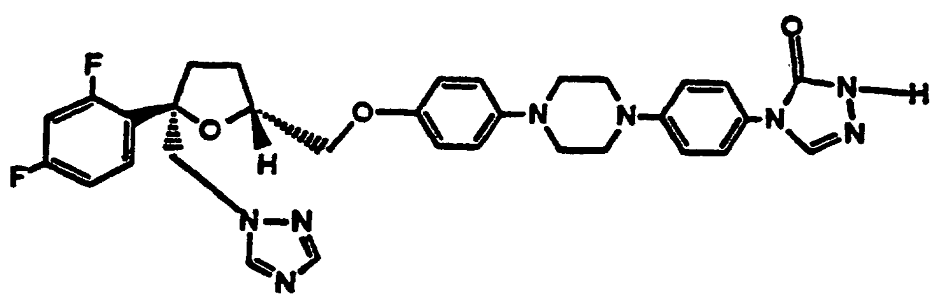

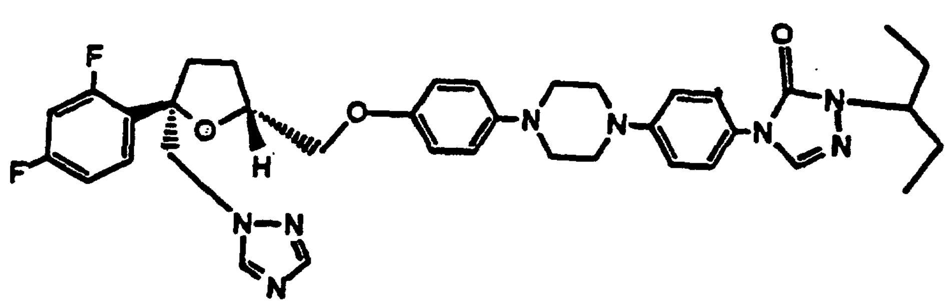

and

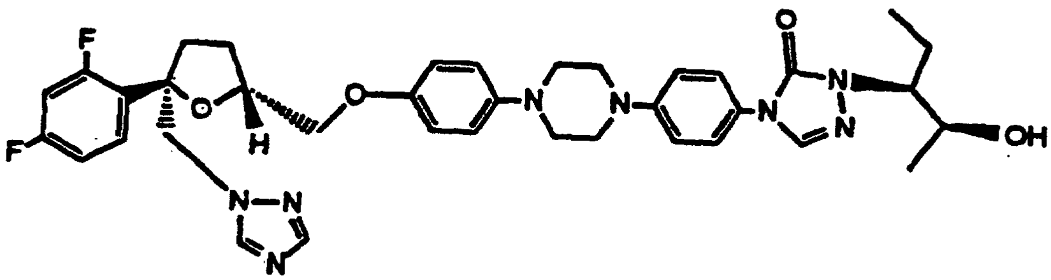

or a pharmaceutically acceptable salt thereof.

**3.** A compound represented by a formula selected from the group consisting of

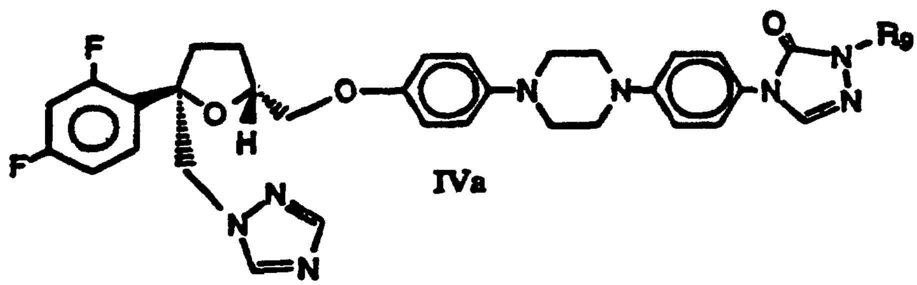

IVa

wherein $R_9$ =

$$-\overset{*}{C}H(C_2H_5)CH(R_6)CH_3 \text{ or } -\overset{*}{C}H(CH_3)CH(R_6)CH_3$$

wherein $R_6$ is OH, or an ester thereof or a pharmaceutically acceptable salt thereof, wherein the ester is a (a) polyether ester (b) phosphate ester (c) heterocyclic ester (d) alkanoate and alkenoate ester (e) amino-acid ester (f) carboxylic acid ester (g) carbonic and carbamic acid ester or (h) sulfate ester.

**4.** The compound according to claim 2 of the formula

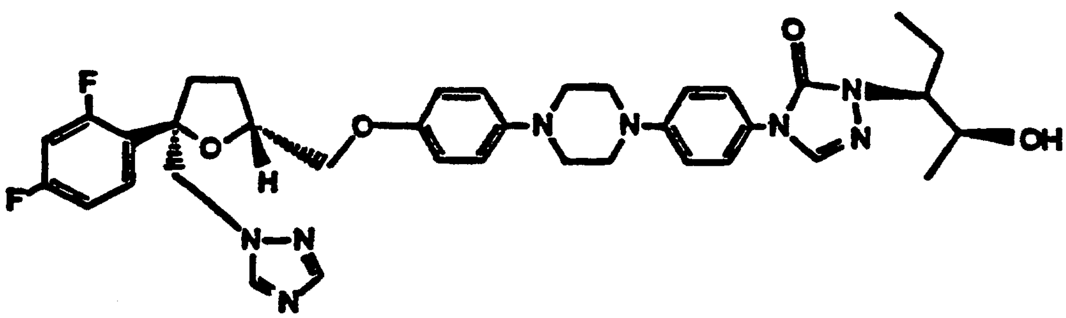

or a pharmaceutically acceptable salt thereof.

**5.** A pharmaceutical composition for treating or preventing fungal infection comprising an antifungally effective amount of a compound of claim 1 together with a pharmaceutically acceptable carrier therefor.

**6.** The use of a compound according to any one of the preceding claims for the manufacture of a medicament for treating and/or preventing fungal infections in a mammal.

**7.** The use of claim 6 wherein the mode of administration is oral or parenteral.

**8.** An amino acid ester salt represented by a formula selected from the group consisting of:

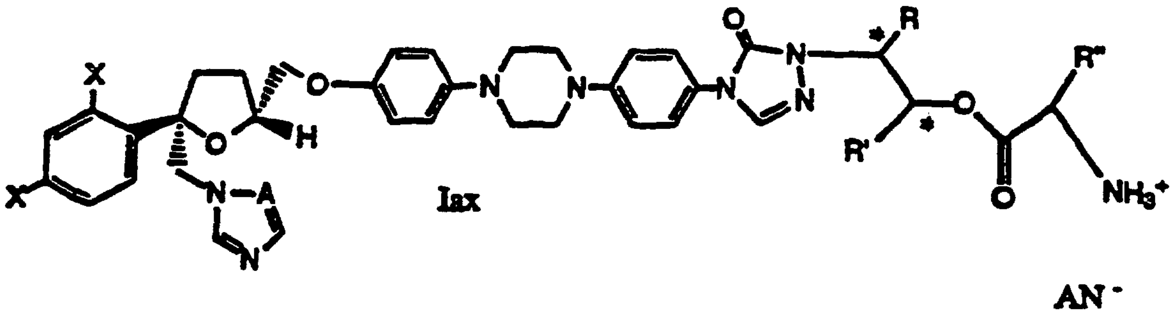

Iax

wherein X is independently selected from the group consisting of F, Cl Br, and $CF_3$;

A is N or CH;
R is H, $CH_3$, or $C_2H_5$,
R' is H, $CH_3$, $C_2H_5$,
$AN^-$ is an anion;
R" is H, $CH_3$, $CH_2OR'''$, $CH(OR''')CH_3$; $CH_2SR'''$,

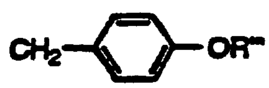
,

$CH_2CONH_2$, $CH_2CH_2,CONH_2$.

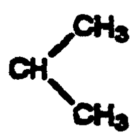
,

$CH(CH_3)CH_2CH_3$,

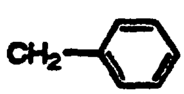
,
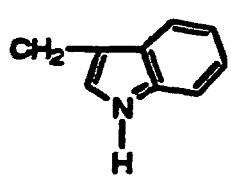
,

$CH_2CH_2SCH_3$,

$CH_2COO^-M^+$, $CH_2CH_2COO^-$ $M^+$, $CH_2CH_2CH_2CH_2NH_3{}^+$ $AN^-$ ,

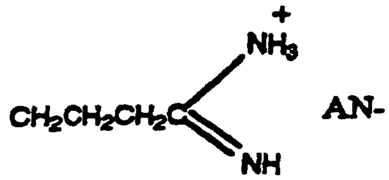 , 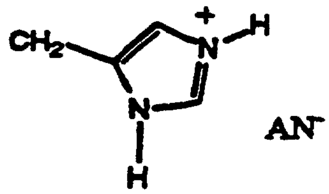

$M^+$ is a metal cation; and
R''' is H, phosphate ester, sulphate ester and proline ester.

**Patentansprüche**

1. Verbindung, die durch eine Formel ausgewählt aus der Gruppe bestehend aus

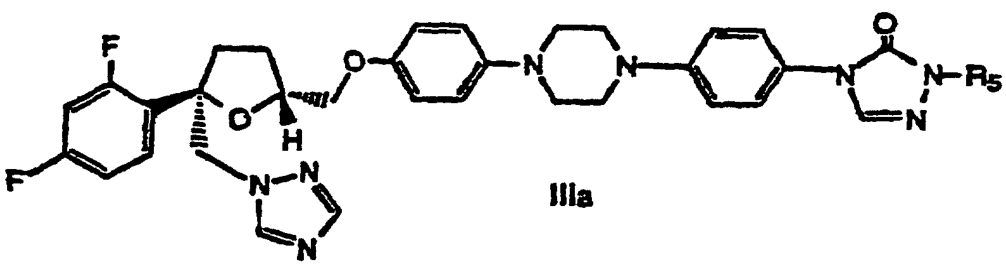

IIIa

dargestellt wird, wobei $R_5$

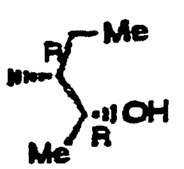 , 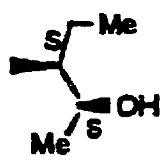 , 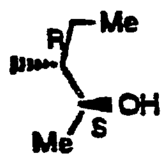 ,

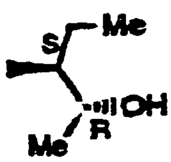 , 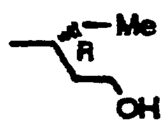 , 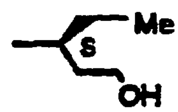 ,

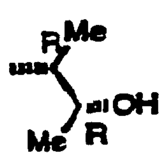 , 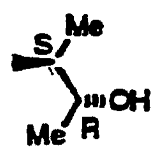 , 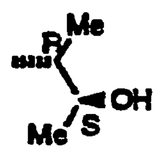 ,

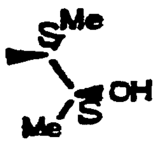 , 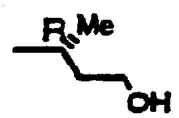 , 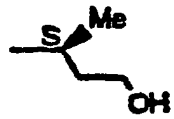 ,

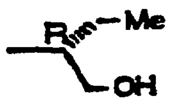 oder 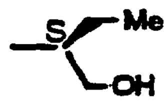

ist, oder ein Ester davon oder ein pharmazeutisch akzeptables Salz davon, wobei der Ester ein (a) Polyetherester, (b) Phosphatester, (c) heterocyclischer Ester, (d) Alkanoat- und Alkenoatester, (e) Aminosäureester, (f) Carbonsäureester, (g) Carbon- und Carbaminsäureester oder (h) Sulfatester ist.

**2.** Verbindung ausgewählt aus der Gruppe bestehend aus

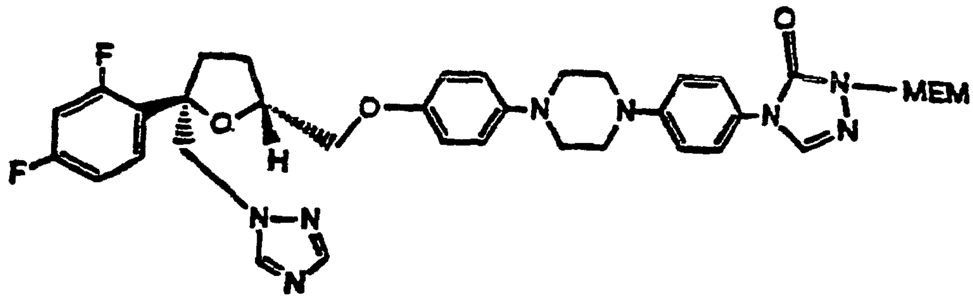

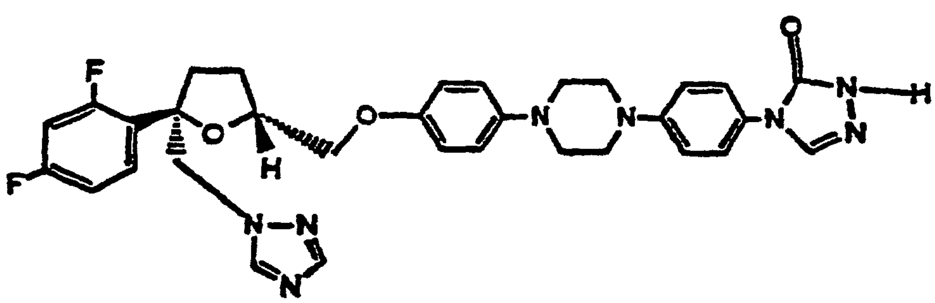

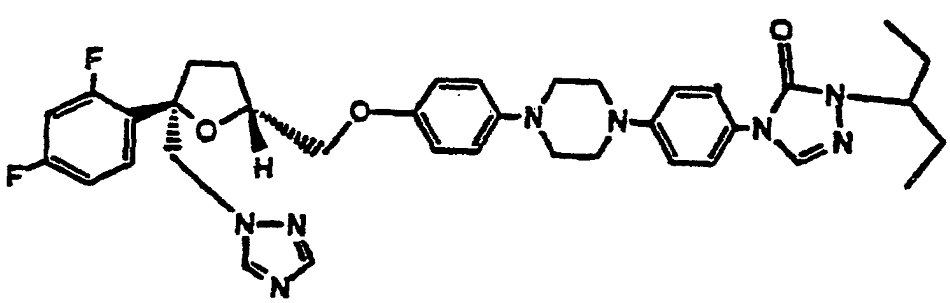

und

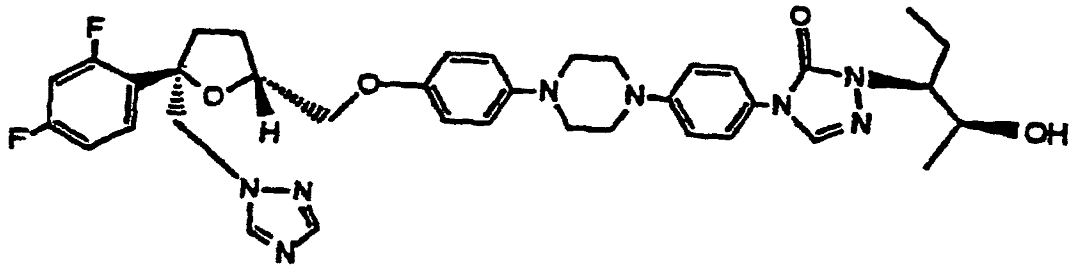

oder ein pharmazeutisch akzeptables Salz davon.

**3.** Verbindung, die durch eine Formel ausgewählt aus der Gruppe bestehend aus

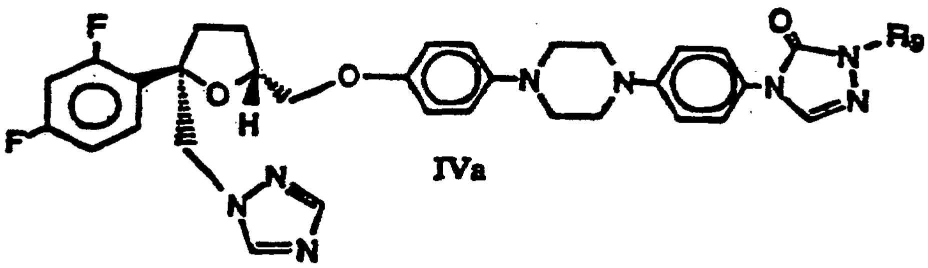

IVa

dargestellt wird,
wobei

$$R_9 = -\overset{*}{C}H(C_2H_5)CH(R_6)CH_3$$

oder

$$-\overset{*}{C}H(CH_3)CH(R_6)CH_3$$

ist, wobei $R_6$ OH oder ein Ester davon oder ein pharmazeutisch akzeptables Salz davon ist, wobei der Ester ein (a) Polyetherester, (b) Phosphatester, (c) heterocyclischer Ester, (d) Alkanoatund Alkenoatester, (e) Aminosäureester, (f) Carbonsäureester, (g) Carbon- und Carbaminsäureester oder (h) Sulfatester ist.

**4.** Verbindung nach Anspruch 2 der Formel

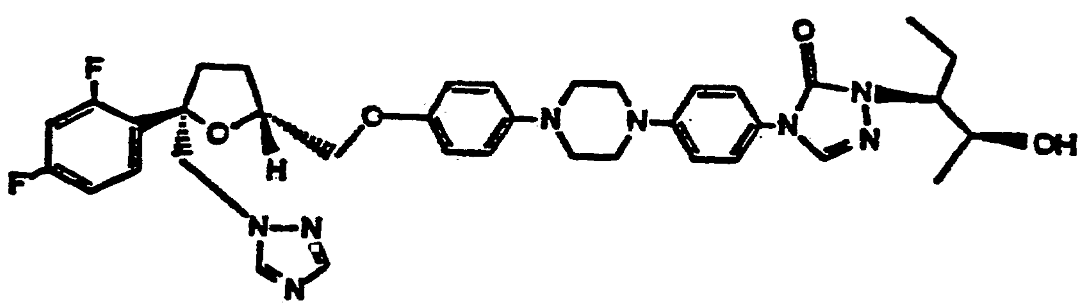

oder ein pharmazeutisch akzeptables Salz davon.

**5.** Pharmazeutische Zusammensetzung zur Behandlung von oder Vorbeugung vor Pilzinfektion, die eine antifungal wirksame Menge einer Verbindung gemäß Anspruch 1 zusammen mit einem pharmazeutisch akzeptablen Träger dafür enthält.

**6.** Verwendung einer Verbindung gemäß einem der vorherigen Ansprüche zur Herstellung eines Medikaments zur Behandlung von und/oder Vorbeugung vor Pilzinfektionen in einem Säugetier.

**7.** Verwendung nach Anspruch 6, wobei die Art der Verabreichung oral oder parenteral ist.

**8.** Aminosäureestersalz, das durch eine Formel ausgewählt aus der Gruppe bestehend aus

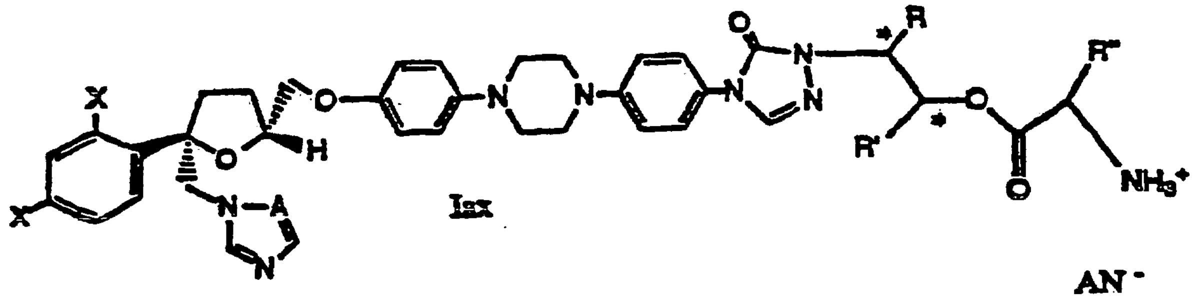

Iαx

dargestellt wird, wobei X unabhängig ausgewählt ist aus der Gruppe bestehend aus F, Cl, Br und $CF_3$;

A N oder CH ist;
R H, $CH_3$ oder $C_2H_5$ ist,
R' H, $CH_3$, $C_2H_5$ ist,
$AN^-$ ein Anion ist;
R" H, $CH_3$, $CH_2OR'''$, $CH((OR''')CH_3$; $CH_2SR'''$

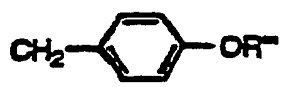,

$CH_2CONH_2$, $CH_2CH_2,CONH_2$,

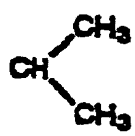,

$CH(CH_3)CH_2CH_3$,

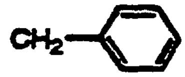, 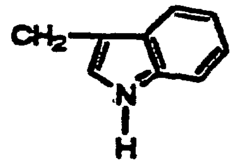,

$CH_2CH_2SCH_3$,

$CH_2COO^- M^+$, $CH_2CH_2COO^- M^+$, $CH_2CH_2CH_2CH_2NH_3^+ AN^-$

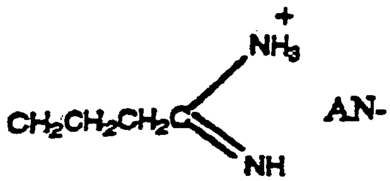

,

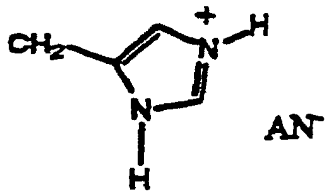

ist,
$M^+$ ein Metallkation ist; und
R''' H, Phosphatester, Sulfatester und Prolinester ist.

## Revendications

**1.** Composé représenté par la formule :

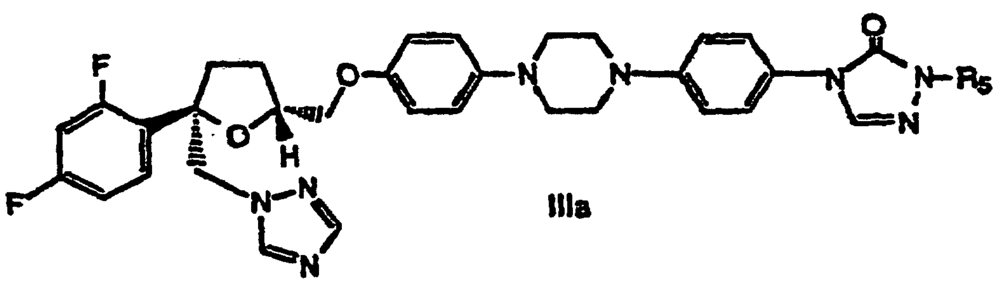

IIIa

dans laquelle $R_5$ représente

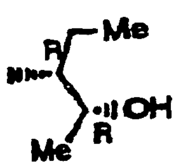

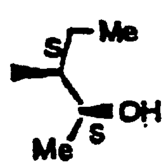

,

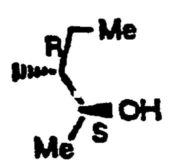

,

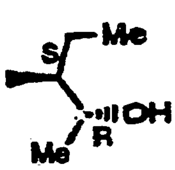

,

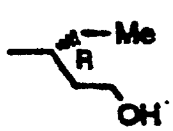

,

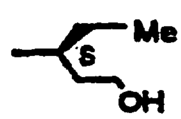

,

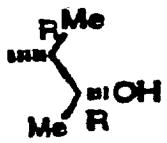 , 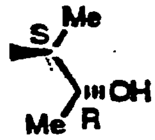 , 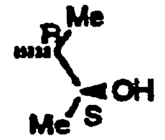 ,

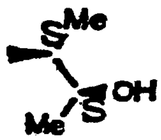 , 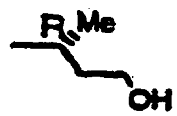 , 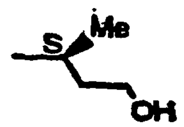 ,

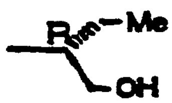 ~~or~~ ou 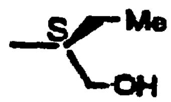

ou un ester ou un sel pharmaceutiquement acceptable d'un tel composé, l'ester étant (a) un ester polyéther, (b) un ester phosphate, (c) un ester hétérocyclique, (d) un ester alcanoate ou alcénoate, (e) un ester d'amino-acide, (f) un ester d'acide carboxylique, (g) un ester d'acide carbonique ou carbamique, ou (h) un ester sulfate.

**2.** Composé choisi parmi les composés suivants :

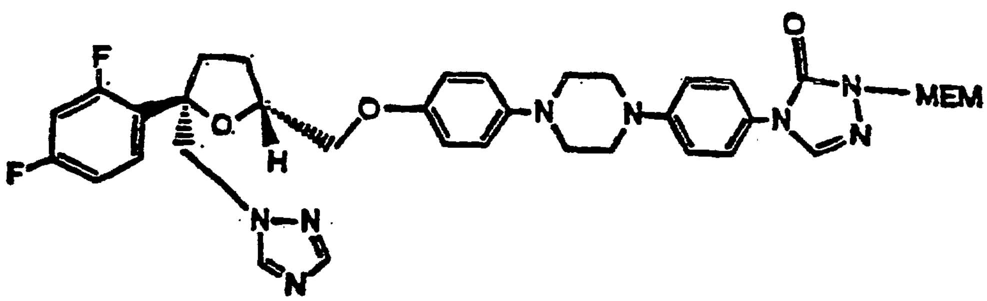

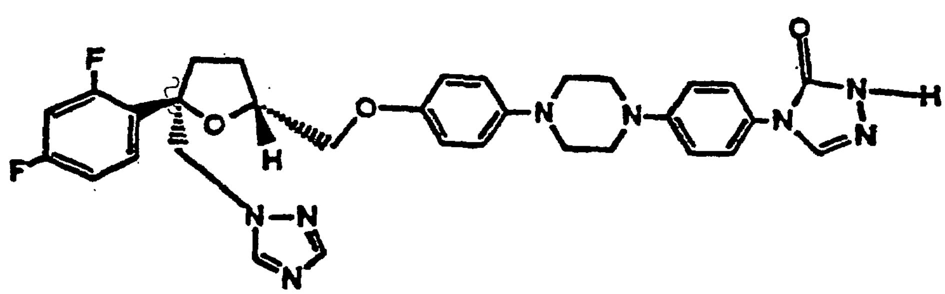

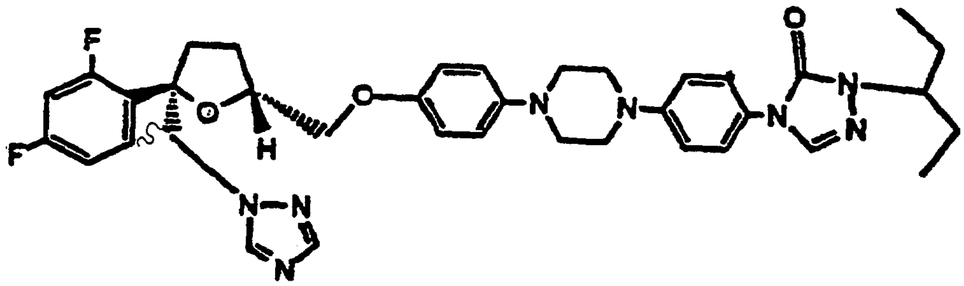

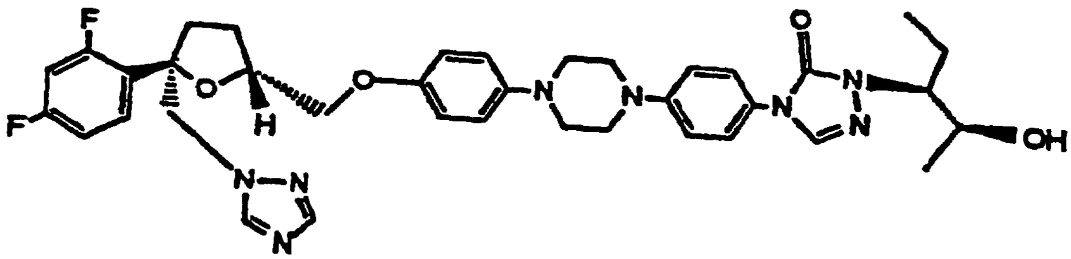

ou un sel pharmaceutiquement acceptable d'un tel composé.

**3.** Composé représenté par la formule :

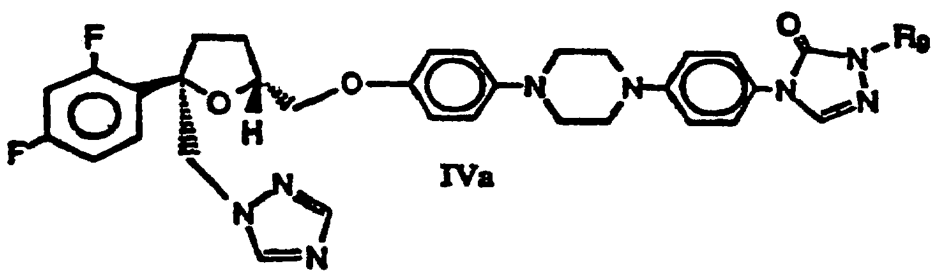

IVa

dans laquelle $R_9$ représente un groupe

$$-\overset{*}{C}H(C_2H_5)CH(R_6)CH_3$$

ou

$$-\overset{*}{C}H(CH_3)CH(R_6)CH_3$$

dans lequel $R_6$ représente OH,
ou un ester ou un sel pharmaceutiquement acceptable d'un tel composé, l'ester étant (a) un ester polyéther, (b) un ester phosphate, (c) un ester hétérocyclique, (d) un ester alcanoate ou alcénoate, (e) un ester d'amino-acide, (f) un ester d'acide carboxylique, (g) un ester d'acide carbonique ou carbamique, ou (h) un ester sulfate.

**4.** Composé selon la revendication 2, de formule :

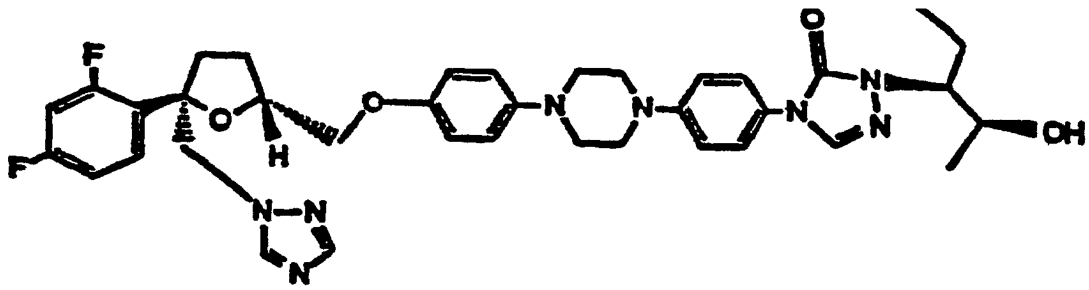

ou un sel pharmaceutiquement acceptable de ce composé.

**5.** Composition pharmaceutique pour le traitement ou la prévention des infections fongiques, qui comprend une quantité efficace quant à l'effet fongicide d'un composé selon la revendication 1, et un support pharmaceutiquement acceptable pour ce composé.

**6.** Utilisation d'un composé selon l'une quelconque des revendications précédentes pour la préparation d'un médicament destiné au traitement et/ou à la prévention des infections fongiques chez un mammifère.

**7.** Utilisation selon la revendication 6, dans laquelle le mode d'administration est oral ou parentéral.

**8.** Sel d'ester d'amino-acide qui est représenté par la formule :

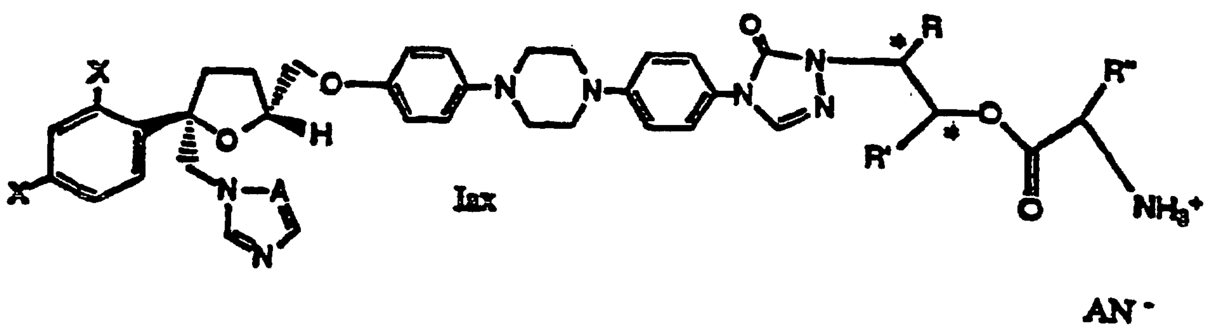

Iax

dans laquelle chaque X représente indépendamment F, Cl, Br ou $CF_3$,

A représente N ou CH,
R représente H, $CH_3$ ou $C_2H_5$,
R' représente H, $CH_3$ ou $C_2H_5$,
$AN^-$ représente un anion,
R" représente

H, $CH_3$, $CH_2OR'''$, $CH(OR''')CH_3$, $CH_2SR'''$,

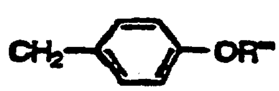
,

$CH_2CONH_2$, $CH_2CH_2,CONH_2$,

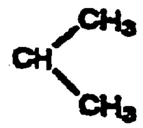,

$CH(CH_3)CH_2CH_3$,

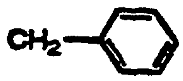, 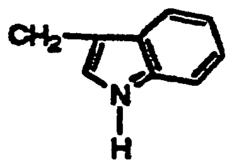,

$CH_2CH_2SCH_3$,

$CH_2COO^- M^+$, $CH_2CH_2COO^- M^+$, $CH_2CH_2CH_2CH_2NH_3{+} AN^-$

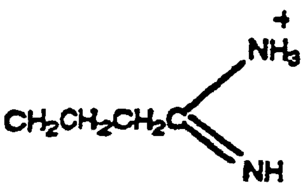 $AN^-$ 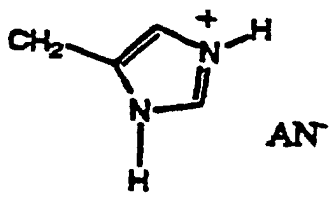 $AN^-$

$M^+$ représente un cation métallique, et
R''' représente H, un groupe ester phosphate, un groupe ester sulfate ou un groupe ester proline.